# EUROPEAN PATENT SPECIFICATION

(11) **EP 1 694 833 B1**
(45) Date of publication and mention of the grant of the patent: **26.08.2009**
(21) Application number: 04798124.6
(22) Date of filing: 30.11.2004
(51) Int. Cl.: C12N 9/10, C12Q 1/48

(54) **2-METHYL-6-SOLANYLBENZOQUINONE METHYLTRANSFERASE AS TARGET FOR HERBICIDES**
2-METHYL-6-SOLANYLBENZOCHINON-METHYLTRANSFERASE ALS ZIEL FÜR HERBIZIDE
2-METHYL-6-SOLANYLBENZOQUINONE METHYLTRANSFERASE UTILISEE COMME CIBLE POUR DES HERBICIDES

(30) Priority: 02.12.2003 DE 10356631
(43) Date of publication of application: 30.08.2006
(73) Proprietor: BASF SE, 67056 Ludwigshafen (DE)
(72) Inventor: EHRHARDT, Thomas, 67346 Speyer (DE); REINDL, Andreas, 68199 Mannheim (DE); FREUND, Annette, 67177 Limburgerhof (DE); SCHMIDT, Ralf-Michael, 67489 Kirrweiler (DE); SONNEWALD, Uwe, 06484 Quedlinburg (DE); STITT NIGEL, Marc, 14469 Potsdam (DE); LEIN, Wolfgang, 14471 Potsdam (DE); BÖRNKE, Frederik, 06484 Quedlinburg (DE)
(74) Representative: Fitzner, Ulrich
(86) International application number: PCT/EP2004/013560
(87) International publication number: WO 2005/054453

(56) References cited:
- WO-A-01/04330
- WO-A-03/034812
- CHENG ZIGANG ET AL: "Highly divergent methyltransferases catalyze a conserved reaction in tocopherol and plastoquinone synthesis in cyanobacteria and photosynthetic eukaryotes." PLANT CELL, vol. 15, no. 10, October 2003 (2003-10), pages 2343-2356, XP002319368 ISSN: 1040-4651 cited in the application
- MOTOHASHI REIKO ET AL: "Functional analysis of the 37 kDa inner envelope membrane polypeptide in chloroplast biogenesis using a Ds-tagged Arabidopsis pale-green mutant." PLANT JOURNAL, vol. 34, no. 5, June 2003 (2003-06), pages 719-731, XP002319369 ISSN: 0960-7412 cited in the application
- ABELL ET AL: "biochemical aproaches to herbicide discovery: advances in enzyme target identification and inhibitor design" WEED SCIENCE, WEED SCIENCE SOCIETY OF AMERICA, CHAMPAIGN, IL, US, vol. 44, 1996, pages 734-742, XP002094170 ISSN: 0043-1745
- DATABASE EMBL APG1 Arabidopsis thaliana 1 December 2001 (2001-12-01), MOTOHASHI ET AL.: XP002319370 retrieved from EBI accession no. Q94IE2
- DATABASE EMBL 37kDa chloroplast inner membrane polypeptide 1 November 1996 (1996-11-01), BLOCK ET AL.: XP002319371 retrieved from EBI accession no. Q40501
- DATABASE EMBL N. tabacum mRNA for 37 kDa protein 30 June 1996 (1996-06-30), BLOCK ET AL.: XP002319372 retrieved from EBI accession no. X94968
- DATABASE EMBL A. thaliana mRNA for APG1 17 July 2001 (2001-07-17), MOTOHASHI ET AL.: XP002319373 retrieved from EBI accession no. AB054257

## Description

The present invention relates to the use of a truncated 2-methyl-6-solanylbenzoquinone methyltransferase which, when absent, brings about retarded growth and chlorotic leaves, as target for herbicides. In this context, novel nucleic acid sequences comprising SEQ ID NO:5 and SEQ ID NO:7 and corresponding functional equivalents are provided. Moreover, the present invention relates to the use of a truncated 2-methyl-6-solanylbenzoquinone methyltransferase and of its functional equivalents in a method for identifying herbicidal or growth-regulatory compounds, and to the use of the compounds identified via the abovementioned method as herbicides or growth regulators.

The basic principle of identifying herbicides via the inhibition of a defined target is known (for example US 5,187,071, WO 98/33925, WO 00/77185, Abell et al., 1996, Weed Science 44:734-742). In general, there is a great demand for the detection of enzymes which might constitute novel targets for herbicides. Reasons for this are resistance problems which arise for herbicides acting on known targets, and the ongoing endeavor to identify novel herbicidal active ingredients which are distinguished by as wide as possible a spectrum of action, ecological and toxicological acceptability and low application rates.

In practice, the detection of novel targets entails great difficulties since the inhibition of an enzyme which forms part of a metabolic pathway frequently has no further effect on the growth of the plant. This may be attributed to the fact that the plant switches to alternative metabolic pathways whose existence is not known or that the inhibited enzyme is not limiting for the metabolic pathway. Moreover, plant genomes are characterized by extensive functional redundancy. Functionally equivalent enzymes are more frequently found in gene families in the genome of Arabidopsis thaliana than in insects or mammals (Nature, 2000, 408(6814):796-815). This theory is confirmed experimentally by the fact that extensive gene knock-out programs in Arabidopsis, which involve the insertion of T-DNA or transposons, have as yet yielded fewer manifested phenotypes than expected (Curr. Op. Plant Biol. 4, 2001, pp.111-117).

It is therefore an object of the present invention to identify novel targets which are essential for the growth of plants and/or whose inhibition leads to a reduced growth of the plant, and to provide methods which are suitable for identifying herbicidal and/or growth-regulatory compounds.

We have found that this object is achieved by the use of a polypeptide with the activity of a 2-methyl-6-solanylbenzoquinone methyltransferase in a method for identifying herbicides.

Some terms used in the description are now defined at this point.

"Affinity tag": this refers to a peptide or polypeptide whose coding nucleic acid sequence can be fused to the nucleic acid sequence of the invention either directly or by means of a linker, using customary cloning techniques. The affinity tag serves for the isolation, concentration and/or selective purification of the recombinant target protein by means of affinity chromatography from total cell extracts. The abovementioned linker can advantageously contain a protease cleavage site (for example for thrombin or factor Xa), whereby the affinity tag can be cleaved from the target protein when required. Examples of common affinity tags are the "His tag", for example from Quiagen, Hilden, "Strep tag", the "Myc tag" (Invitrogen, Carlsberg), the tag from New England Biolabs which consists of a chitin-binding domain and an inteine, the maltose-binding protein (pMal) from New England Biolabs, and what is known as the CBD tag from Novagen. In this context, the affinity tag can be attached to the 5' or the 3' end of the coding nucleic acid sequence with the sequence encoding the target protein.

"Activity": the term enzymatic activity describes the ability of an enzyme to convert a substrate into a product. The activity can be determined via the increase in the product, the decrease in the substrate (or starting material) or the decrease in a specific cofactor, or via a combination of at least two of the abovementioned parameters, as a function of a defined period of time.

"Expression cassette": an expression cassette contains a nucleic acid sequence according to the invention linked operably to at least one genetic control element, such as a promoter, and, advantageously, to a further control element, such as a terminator. The nucleic acid sequence of the expression cassette can be for example a genomic or complementary DNA sequence or an RNA sequence, and their semisynthetic or fully synthetic analogs. These sequences can exist in linear or circular form, extrachromosomally or integrated into the genome. The nucleic acid sequences in question can be synthesized or obtained naturally or contain a mixture of synthetic and natural DNA components, or else consist of various heterologous gene segments of various organisms.

Artificial nucleic acid sequences are also suitable in this context as long as they make possible the expression, in a cell or an organism, of a polypeptide encoded by a nucleic acid sequence according to the invention and with the activity of the 2-methyl-6-solanylbenzoquinone methyltransferase. For example, synthetic nucleotide sequences can be generated which have been optimized with regard to the codon usage of the organisms to be transformed.

All of the abovementioned nucleotide sequences can be generated from the nucleotide units by chemical synthesis in a manner known per se, for example by fragment condensation of individual overlapping complementary nucleotide units of the double helix. Oligonucleotides can be synthesized chemically for example in a manner known per se using the phosphoamidite method (Voet, Voet, 2^{nd} Edition, Wiley Press New York, pp. 896-897). When preparing an expression cassette, various DNA fragments can be manipulated in such a way that a nucleotide sequence with the correct direction of reading and the correct reading frame is obtained. The nucleic acid fragments are linked with each other via general cloning techniques as are described, for example, in T. Maniatis, E.F. Fritsch and J. Sambrook, "Molecular Cloning: A Laboratory Manual", Cold Spring Harbor Laboratory, Cold Spring Harbor, NY (1989), in T.J. Silhavy, M.L. Berman and L.W. Enquist, Experiments with Gene Fusions, Cold Spring Harbor Laboratory, Cold Spring Harbor, NY (1984) and in Ausubel, F.M. et al., "Current Protocols in Molecular Biology", Greene Publishing Assoc. and Wiley-Interscience (1994).

"Operable linkage": an operable, or functional, linkage is understood as meaning the sequential arrangement of regulatory sequences or genetic control elements in such a way that each of the regulatory sequences, or each of the genetic control elements, can fulfill its intended function when the coding sequence is expressed.

"Functional equivalents" describe, in the present context, nucleic acid sequences which hybridize under standard conditions with a nucleic acid sequence or part of a nucleic acid sequence and which are capable of bringing about, in a cell or organism, the expression of a 2-methyl-6-solanylbenzoquinone methyltransferase.

To carry out the hybridization, it is advantageous to use short oligonucleotides with a length of approximately 10-50 bp, preferably 15-40 bp, for example of the conserved or other regions, which can be determined in the manner with which the skilled worker is familiar by comparisons with other related genes. However, longer fragments of the nucleic acids according to the invention with a length of 100-500 bp, or the complete sequences, may also be used for hybridization. Depending on the nucleic acid/oligonucleotide used, the length of the fragment or the complete sequence, or depending on which type of nucleic acid, i.e. DNA or RNA, is being used for the hybridization, these standard conditions vary. Thus, for example, the melting temperatures for DNA:DNA hybrids are approximately 10°C lower than those of DNA:RNA hybrids of the same length.

Standard hybridization conditions are to be understood as meaning, depending on the nucleic acid, for example temperatures of between 42 and 58°C in an aqueous buffer solution with a concentration of between 0.1 and 5 x SSC (1 x SSC = 0.15 M NaCl, 15 mM sodium citrate, pH 7.2) or additionally in the presence of 50% formamide, such as, for example, 42°C in 5 x SSC, 50% formamide. The hybridization conditions for DNA:DNA hybrids are advantageously 0.1 x SSC and temperatures of between approximately 20°C and 65°C, preferably between approximately 30°C and 45°C. In the case of DNA:RNA hybrids, the hybridization conditions are advantageously 0.1 x SSC and temperatures of between approximately 30°C and 65°C, preferably between approximately 45°C and 55°C. These hybridization temperatures which have been stated are melting temperature values which have been calculated by way of example for a nucleic acid with a length of approx. 100 nucleotides and a G + C content of 50% in the absence of formamide. The experimental conditions for DNA hybridization are described in relevant textbooks of genetics such as, for example, in Sambrook et al., "Molecular Cloning", Cold Spring Harbor Laboratory, 1989, and can be calculated using formulae with which the skilled worker is familiar, for example as a function of the length of the nucleic acids, the type of the hybrids or the G + C content. The skilled worker will find further information on hybridization in the following textbooks: Ausubel et al. (eds.), 1985, "Current Protocols in Molecular Biology", John Wiley & Sons, New York; Hames and Higgins (eds.), 1985, "Nucleic Acids Hybridization: A Practical Approach", IRL Press at Oxford University Press, Oxford; Brown (ed.), 1991, "Essential Molecular Biology: A Practical Approach", IRL Press at Oxford University Press, Oxford.

A functional equivalent is understood as meaning furthermore also nucleic acid sequences which are up to a defined percentage homologous or identical with a certain nucleic acid sequence ("original nucleic acid sequence") and which have the same activity as the original nucleic acid sequences, furthermore in particular also natural or artificial mutations of these nucleic acid sequences. Corresponding definitions can be found at suitable places of the description.

The term "functional equivalent" furthermore also encompasses by means of the present invention those nucleotide sequences which are obtained by modification of the nucleic acid sequences SEQ ID NO:5 or SEQ ID NO:7. For example, such modifications can be generated by techniques with which the skilled worker is familiar, such as "Site Directed Mutagenesis", "Error Prone PCR", "DNA shuffling" (Nature 370, 1994, pp.389-391) or "Staggered Extension Process" (Nature Biotechnol. 16, 1998, pp. 258-261). The aim of such a modification can be, for example, the insertion of further cleavage sites for restriction enzymes, the removal of DNA in order to truncate the sequence, the substitution of nucleotides to optimize the codons, or the addition of further sequences. Proteins which are encoded via modified nucleic acid sequences must retain the desired functions despite a deviating nucleic acid sequence.

Functional equivalents thus comprise naturally occurring variants of the herein-described sequences and artificial nucleic acid sequences, for example those which have been obtained by chemical synthesis and which are adapted to the codon usage, and also the amino acid sequences derived from them.

"Genetic control sequence" describes sequences which have an effect on the transcription and, if appropriate, translation of the nucleic acids according to the invention in prokaryotic or eukaryotic organisms. Examples thereof are promoters, terminators or what are known as "enhancer" sequences. In addition to these control sequences, or instead of these sequences, the natural regulation of these sequences may still be present before the actual structural genes and may, if appropriate, have been genetically modified in such a way that the natural regulation has been switched off and the expression of the target gene has been modified, that is to say increased or reduced. The choice of the control sequence depends on the host organism or starting organism. Genetic control sequences furthermore also comprise the 5'-untranslated region, introns or the noncoding 3' region of genes. Control sequences are furthermore understood as meaning those which make possible homologous recombination or insertion into the genome of a host organism or which permit removal from the genome. Genetic control sequences also comprise further promoters, promoter elements or minimal promoters, and sequences which affect chromotin structure (for example matrix attachment regions (MARs)) which are capable of modifying the expression-controlling properties. Owing to genetic control sequences, tissue-specific expression, for example, can thus additionally take place as a function of certain stress factors. Such elements are described, for example, for water stress, abscisic acid (Lam E and Chua NH, J Biol Chem 1991; 266(26): 17131 -17135), chilled and dry stress (Plant Cell 1994, (6): 251-264) and heat stress (Molecular & General Genetics, 1989, 217(2-3): 246-53).

"Homology" between two nucleic acid sequences or polypeptide sequences is defined by the identity of the nucleic acid sequence/polypeptide sequence over in each case the entire sequence length, which is calculated by alignment with the aid of the BESTFIT alignment (Needleman and Wunsch 1970, J. Mol. Biol. 48; 443-453), setting the following parameters for amino acids:

| | |
|---|---|
| Gap Weight: 8 | Length Weight: 2 |
| Average Match: 2,912 and the following parameters for nucleic acids | Average Mismatch: -2,003 |

| | |
|---|---|
| Gap Weight: 50 | Length Weight: 3 |
| Average Match: 10,000 | Average Mismatch: 0,000 |

In the following text, the term "identity" is also used synonymously with the term "homologous" or "homology".

"Mutations" of nucleic or amino acid sequences comprise substitutions, additions, deletions, inversions or insertions of one or more nucleotide residues, which may also bring about changes in the corresponding amino acid sequence of the target protein by substitution, insertion or deletion of one or more amino acids, but with the totality of the functional properties of the target gene being essentially retained.

"Natural genetic environment" means the natural chromosomal locus in the organism of origin. In the case of a genomic library, the natural genetic environment of the nucleic acid sequence is preferably retained at least in part. The environment flanks the nucleic acid sequence at least at the 5' or 3' side and has a sequence length of at least 50 bp, preferably at least 100 bp, especially preferably at least 500 bp, very especially preferably at least 1000 bp, and most preferably at least 5000 bp.

"Plants" for the purposes of the invention are plant cells, plant tissues, plant organs or intact plants, such as seeds, tubers, flowers, pollen, fruits, seedlings, roots, leaves, stalks or other plant parts. Plants are furthermore understood as meaning propagation material such as seeds, fruits, seedlings, sets, tubers, cuttings or root stocks.

In the present context, "2-methyl-6-solanylbenzoquinone" is synonymous with 2-methyl-6-(4,8,12,16,20,24,28,32,36-nonamethyl-heptatriaconta-3,7,11,15,19,23,27,31,35-nonaenyl)benzene-1,4-diol.

In the present context, "2-methyl-6-solanylbenzoquinol" is synonymous with 2-methyl-6-(4,8,12,16,20,24,28,32,36-nonamethyl-heptatriaconta-3,7,11,15,19,23,27,31,35-nonaenyl)-[1,4]benzoquinone.

In the present context, "plastoquinone" is synonymous with 2,3-dimethyl-5-(4,8,12,16,20,24,28,32,36-nonamethyl-heptatriaconta-3,7,11,15,19,23,27,31,35-nonaenyl)-benzene-[1,4]-benzoquinone.

In the present context, "plastoquinol" is synonymous with 2,3-dimethyl-5-(4,8,12,16,20,24,28,32,36-nonamethyl-heptatriaconta-3,7,11,15,19,23,27,31,35-nonaenyl)benzene-1,4-diol.

In the present context, "polypeptide with the activity of 2-methyl-6-solanylbenzoquinone methyltransferase" or 2-methyl-6-solanylbenzoquinol methyltransferase refers to an enzyme which is capable of methylating 2-methyl-6-solanylbenzoquinol to plastoquinol or 2-methyl-6-solanylbenzoquinone to plastoquinone.

"Reaction time" refers to the time required for carrying out an enzyme activity assay until a significant finding is obtained; it depends both on the specific activity of the protein employed in the assay and on the method used and the sensitivity of the instruments used. The skilled worker is familiar with the determination of the reaction times. In the case of methods for identifying herbicidally active compounds which are based on photometry, the reaction times are generally between > 0 and 720 minutes. "Recombinant DNA" describes a combination of DNA sequences which can be generated by recombinant DNA technology.

"Recombinant DNA technology": generally known techniques for fusing DNA sequences (for example described in Sambrook et al., 1989, Cold Spring Harbor, NY, Cold Spring Harbor Laboratory Press).

"Replication origins" ensure the multiplication of the expression cassettes or vectors according to the invention in microorganisms and yeasts, for example the pBR322 ori, or the P15A ori in E. coli (Sambrook et al.: "Molecular Cloning. A Laboratory Manual", 2nd ed., Cold Spring Harbor Laboratory Press, Cold Spring Harbor, NY, 1989) and the ARS1 ori in yeast (Nucleic Acids Research, 2000, 28(10): 2060-2068).

"Reporter genes" encode readily quantifiable proteins. The transformation efficacy or the expression site or timing can be assessed by means of these genes via growth assay, fluorescence assay, chemoluminescence assay, bioluminescence assay or resistance assay or via a photometric measurement (intrinsic color) or enzyme activity. Very especially preferred in this context are reporter proteins (Schenborn E, Groskreutz D, Mol. Biotechnol. 1999; 13(1):29-44) such as the "green fluorescent protein" (GFP) (Gerdes HH and Kaether C, FEBS Lett. 1996; 389(1):44-47; Chui WL et al., Curr. Biol 1996, 6:325-330; Leffel SM et al., Biotechniques 23(5):912-8, 1997), chloramphenicol acetyl transferase, a luciferase (Giacomin, Plant Sci 1996, 116:59-72; Scikantha, J. Bact. 1996, 178:121; Millar et al., Plant Mol. Biol. Rep. 1992 10:324-414), and luciferase genes, in general β-galactosidase or β-glucuronidase (Jefferson et al., EMBO J. 1987, 6, 3901-3907) or the Ura3 gene.

"Selection markers" confer resistance to antibiotics or other toxic compounds: examples which may be mentioned in this context are the neomycin phosphotransferase gene, which confers resistance to the aminoglycoside antibiotics neomycin (G 418), kanamycin, paromycin (Deshayes A et al., EMBO J. 4 (1985) 2731-2737), the sul gene, which encodes a mutated dihydropteroate synthase (Guerineau F et al., Plant Mol. Biol. 1990; 15(1):127-136), the hygromycin B phosphotransferase gene (Gen Bank Accession NO: K 01193) and the shble resistance gene, which confers resistance to the bleomycin antibiotics such as zeocin. Further examples of selection marker genes are genes which confer resistance to 2-deoxyglucose-6-phosphate (WO 98/45456) or phosphinothricin and the like, or those which confer a resistance to antimetabolites, for example the dhfr gene (Reiss, Plant Physiol. (Life Sci. Adv.) 13 (1994) 142-149). Examples of other genes which are suitable are trpB or hisD (Hartman SC and Mulligan RC, Proc. Natl. Acad. Sci. U S A 85 (1988) 8047-8051). Another suitable gene is the mannose phosphate isomerase gene (WO 94/20627), the ODC (ornithine decarboxylase) gene (McConlogue, 1987 in: Current Communications in Molecular Biology, Cold Spring Harbor Laboratory, Ed.) or the Aspergillus terreus deaminase (Tamura K et al., Biosci. Biotechnol. Biochem. 59 (1995) 2336-2338).

"Transformation" describes a process for introducing heterologous DNA into a pro- or eukaryotic cell. A transformed cell describes not only the product of the transformation process per se, but also all of the transgenic progeny of the transgenic organism generated by the transformation.

"Target/target protein": a polypeptide encodes by the nucleic acid sequence according to the invention, which may take the form of an enzyme in the traditional sense, a structural protein, a protein relevant for developmental processes, regulatory proteins such as transcription factors, kinases, phosphatases, receptors, subunits of channels, transport proteins, regulatory subunits which confer substrate or activity regulation on an enzyme complex. All of the targets or sites of action share the characteristic that the functional presence of the target protein is essential for survival or normal development and growth.

"Transgenic": referring to a nucleic acid sequence, an expression cassette or a vector comprising a nucleic acid sequence according to the invention or an organism transformed with a nucleic acid sequence, expression cassette or vector according to the invention, the term "transgenic" describes all those constructs which have been generated by genetic engineering methods in which either the nucleic acid sequence of the target protein or a genetic control sequence linked operably to the nucleic acid sequence of the target protein or a combination of the abovementioned possibilities are not in their natural genetic environment or have been modified by recombinant methods. In this context, the modification can be achieved, for example, by mutating one or more nucleotide residues of the nucleic acid sequence in question.

In the present application, reference is made to the following sequences:

| Name | Organism | Sequence | |
|---|---|---|---|
| SEQ ID NO:1 | N. tabacuum | NA | UT |
| SEQ ID NO:2 | N. tabacuum | AA | UT |
| SEQ ID NO:3 | A. thaliana | NA | UT |
| SEQ ID NO:4 | A. thaliana | AA | UT |
| SEQ ID NO:5 | A. thaliana | NA | C(31)+N (49) |
| SEQ ID NO:6 | A. thaliana | AA | C(31)+N (49) |
| SEQ ID NO:7 | A. thaliana | NA | C(31)+N (51) |
| SEQ ID NO:8 | A. thaliana | AA | C(31)+N (51) |
| SEQ ID NO:9 | N. tabacuum | NA | PS |
| SEQ ID NO:10 | N. tabacuum | AA | PS |

| | | | |
|---|---|---|---|
| *) Nicotiana = N. Arabidopsis= A. AA=amino acid sequence NA= nucleic acid sequence C ()+N () = C- and N-terminally truncated sequence (with regard to SEQ ID NO:3, the truncated AS is stated) UT = untruncated sequence PS = part sequence | | | |

Plastoquinone is an essential cofactor in plant photosynthesis. As a two-electron redox partner, it ensures that electrons are transferred from photosystem II to the cytochrome b₆/f complex, and furthermore acts as cofactor in carotenoid biosynthesis. Plastoquinone biosynthesis branches off from the biosynthesis of the aromatic amino acids. Homogentisate solanyltransferase transfers a solanyl residue to homogentisate. The resulting 2-methyl-6-solanylbenzoquinol (MSBQ) is subsequently methylated by 2-methyl-6-solanylbenzoquinone methyltransferase (MSBQ-MT) to plastoquinol. MSBQ-MT requires S-adenosylmethionine as methyl donor. Moreover, MSBQ-MT is furthermore involved in tocopherol biosynthesis since it accepts not only MSBQ, but also 2-methyl-6-phytylbenzoquinone (MPBQ) as substrate. Both enzymes are located in the chloroplast. Homogentisate is formed in the cytosol by the effect of hydroxyphenylpyruvate dioxygenase (HPPD) on p-hydroxyphenylpyruvate. All enzymes of plastoquinone biosynthesis have by now been cloned from higher plants.

In the course of the evolution, MSBQ-MT enzymes have probably developed independently in cyano bacteria and in higher plants since functional homology, but not sequence homology, exists between these enzymes from cyano bacteria and from higher plants (Cheng et al. 2003 Plant Cell 15, pp. 2343-2356).

The importance of plastoquinone biosynthesis for the fitness of plants is shown with reference to described KO mutants. Arabidopsis HPPD knock-out mutants (pds1) and homogentisate solanyltransferase knock-out mutants (pds2) are seedling-lethal under photoautotrophic conditions and contain no plastoquinone (Norris et al. 1998, Plant Cell 7, pp. 2139-2149). Very similar phenotypes display MSBQ-MT knock-out mutants (vte3-2, Cheng et al. 2003 Plant Cell 15, pp. 2343-2356 and apg1, Motohashi et al. 2003 The Plant Journal 34, pp. 719-731) which do not survive in soil and are pale green and growth-retarded on sucrose-comprising agar media. WO 01/04330 A describes the overexpression of a functional homologue, the 2-Methyl-6-phytylhydroquinone-methyltransferase.

Moreover, HPPD is the site of action of triketone-type herbicides. These data suggest that plant homogentisate solanyltransferase and MSBQ-MT might also be suitable as herbicide target. Inhibitors of these enzymes which are suitable as herbicides have not been disclosed to date.
Cheng et al. 2003 Plant Cell 15, pp. 2343-2356 moreover describe a weak vte3-1 allele. A point mutation in the Arabidopsis MSBQ-MT gene leads to a small decrease of the plastoquinone quantity by 17% compared with the wild type. However, growth and fitness of the vte3-1 mutants are not substantially adversely affected.

Surprisingly, it was possible to demonstrate in the present invention that not only the total loss of the MSBQ-MT activity, as in the zero-mutants vte3-1 and apg1, leads to a negative effect on vitality. It was demonstrated in transgenic tobacco plants which contain an MSBQ-MT antisense gene and which only show partially reduced MSBQ-MT activities that even small decreases in the MSBQ activity lead to drastically reduced vitality. This demonstrates the particular suitability of MSBQ-MT as herbicide target.

A disadvantage regarding the use of MSBQ-MT in an efficient high throughput screening is that an expression in E. coli of the Arabidopsis MSBQ-MT is possible in principle (Cheng et al. 2003 Plant Cell 15, pp. 2343-2356), but only provides very little MSBQ-MT activity, which is insufficient for efficient-throughput screening.

Surprisingly, it has been found within the context of the present invention that N- and C-terminally truncated amino acid sequences of MSBQ-MT can be expressed particularly well in contrast to the corresponding full-length sequences and are thus particularly suitable for the use in assay systems for identifying herbicides.

With reference to nucleic acid sequences, the term "comprising" or "to comprise" means that the nucleic acid sequence according to the invention may comprise, at the 3' and/or at the 5' end, additional nucleic acid sequences, the length of the additional nucleic acid sequences not exceeding 18 bp at the 5' end and 18 bp at the 3' end of the nucleic acid sequences according to the invention, preferably 6 bp at the 5' end and 6 bp at the 3' end, is especially preferably 0 bp at the 5' end and 0 bp at the 3'end.

Furthermore preferred is the use of a 2-methyl-6-solanylbenzoquinone methyltransferase with a amino acid sequence, which is encoded by a nucleic acid sequence as per i), ii) or iii), is truncated at the C terminus by at least 20 amino acids, preferably at least 21-32 amino acids, especially preferably at least 33-48 amino acids, very especially preferably at least 49, 50 or 51 amino acids, and by at least 20 amino acids, preferably at least 21-26 amino acids, especially preferably at least 27-28 amino acids, very especially preferably at least 29, 30 or 31 amino acids, at the N terminus.

Especially preferred in this context is the use of a 2-methyl-6-solanylbenzoquinone methyltransferase whose amino acid sequence is encoded by a nucleic acid sequence comprising
iv) a nucleic acid sequence with the nucleic acid sequence shown in SEQ ID NO:5 or in SEQ ID NO:7; or
v) a nucleic acid sequence which, owing to the degeneracy of the genetic code, can be derived from the amino acid sequence shown in SEQ ID NO:6 or in SEQ ID NO:8 by back translation.

All of the abovementioned nucleic acid sequences originate preferably from a plant.

Furthermore claimed within this context are plant nucleic acid sequences encoding a polypeptide with the activity of a 2-methyl-6-solanylbenzoquinone methyltransferase comprising
i) a nucleic acid sequence with the nucleic acid sequence shown in SEQ ID NO:5 or in SEQ ID NO:7; or
ii) a nucleic acid sequence which, owing to the degeneracy of the genetic code, can be derived from the amino acid sequence shown in SEQ ID NO:6 or in SEQ ID NO: 8 by back translation.

The truncated amino acid sequences encoding a polypeptide with the activity of a 2-methyl-6-solanylbenzoquinone methyltransferase are encoded by
v) a nucleic acid sequence with the nucleic acid sequence shown in SEQ ID NO:5 or in SEQ ID NO:7; or
vi) a nucleic acid sequence which, owing to the degeneracy of the genetic code, can be derived from the amino acid sequence shown in SEQ ID NO:6 or in SEQ ID NO:8 by back translation.

For the sake of simplicity, a polypeptide with the activity of a 2-methyl-6-solanylbenzoquinone methyltransferase and which is encoded by nucleic acid sequences i), ii) or iii) according to the invention is hereinbelow referred to as "MSM". The truncated polypeptides encoded by the nucleic acid sequences iv), v) or vi) according to the invention are hereinbelow referred to as "VMSM". The nucleic acid sequences iv), v) or vi) according to the invention are referred to as "VMSM sequence".

The gene products of the nucleic acids according to the invention are novel targets for herbicides and growth regulators, preferably herbicides, which make possible the provision of novel herbicides for controlling undesired plants.

Undesired plants are understood as meaning in the broadest sense all those plants which grow at locations where they are undesired, for example:
dicotyledonous weeds of the genera: *Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.*
monocotyledonous weeds of the genera: *Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, Apera.*

SEQ ID NO:1, SEQ ID NO:3, SEQ ID NO:5 or SEQ ID NO:7 or parts of the abovementioned nucleic acid sequence can be used for the generation of hybridization probes. The generation of these probes and the experimental procedure are known. For example, this can be effected via the selective generation of radioactive or nonradioactive probes by PCR and the use of suitably labeled oligonucleotides, followed by hybridization experiments. The technologies required for this purpose are detailed, for example, in T. Maniatis, E.F. Fritsch and J. Sambrook, Molecular Cloning: A Laboratory Manual, Cold Spring Harbor Laboratory, Cold Spring Harbor, NY (1989). The probes in question can furthermore be modified by standard technologies (Lit. SDM or random mutagenesis) in such a way that they can be employed for further purposes, for example, as a probe which hybridizes specifically with mRNA and the corresponding coding sequences for analysis of the corresponding sequences in other organisms.

The above probes can be used for the detection and isolation of functional equivalents (see definition above) from other plant species on the basis of sequence identities. For this purpose, for example part or all of the sequence of the nucleic acid sequence in question is used as probe for screening in a genomic library or cDNA library of the plant species in question or in a computer search for sequences of functional equivalents in electronic databases.

Preferred plant species in this context are the undesired plants which have already been mentioned at the outset.

The present invention furthermore relates to expression cassettes comprising
a) genetic control sequences in operable linkage with VMSM sequence;
b) additional functional elements; or
c) a combination of a) and b);
and to the use of expression cassettes comprising
a) genetic control sequences in operable linkage with a nucleic acid sequence according to the invention;
b) additional functional elements; or
c) a combination of a) and b)

with the expression of MSM or VMSM, preferably VMSM, for use in *in vitro* assay systems. Both use forms of the above-described expression cassettes are hereinbelow referred to as the expression cassette according to the invention.

In a preferred embodiment, an expression cassette according to the invention comprises a promoter at the 5' end of the coding sequence and, at the 3' end, a transcription termination signal and, if appropriate, further genetic control sequences which are linked operably with the interposed nucleic acid sequence according to the invention.

The expression cassettes according to the invention are also understood as meaning analogs which can be brought about for example by a combination of the individual nucleic acid sequences on the polynucleotide (multiple constructs), on a plurality of polynucleotides in one cell (cotransformation) or by sequential transformation.

Advantageous genetic control sequences according to point a) for the expression cassettes according to the invention or for vectors comprising them are, for example, promoters such as the cos, tac, trp, tet, lpp, lac, laclq, T7, T5, T3, gal, trc, ara, SP6, λ-PR or the λ-PL promoter, all of which can be used for expressing MSM or VMSM, preferably VMSM, in Gram-negative bacterial strains.

Examples of further advantageous genetic control sequences are present, for example, in the promoters amy and SPO2, both of which can be used for expressing MSM or VMSM, preferably VMSM in Gram-positive bacterial strains, and in the yeast or fungal promoters AUG1, GPD-1, PX6, TEF, CUP1, PGK, GAP1, TPI, PHO5, AOX1, GAL10/CYC1, CYC1, OliC, ADH, TDH, Kex2, MFa or NMT or combinations of the abovementioned promoters (Degryse et al., Yeast 1995 June 15; 11(7):629-40; Romanos et al. Yeast 1992 June;8(6):423-88; Benito et al. Eur. J. Plant Pathol. 104, 207-220 (1998); Cregg et al. Biotechnology (N Y) 1993 Aug;11(8):905-10; Luo X., Gene 1995 Sep 22;163(1):127-31: Nacken et al., Gene 1996 Oct 10;175(1-2): 253-60; Turgeon et al., Mol Cell Biol 1987 Sep;7(9):3297-305) or the transcription terminators NMT, Gcy1, TrpC, AOX1, nos, PGK or CYC1 (Degryse et al., Yeast 1995 June 15; 11(7):629-40; Brunelli et al. Yeast 1993 (Dec9(12): 1309-18; Frisch et al., Plant Mol. Biol. 27(2), 405-409 (1995); Scorer et al., Biotechnology (N.Y. 12 (2), 181-184 (1994), Genbank acc. number Z46232; Zhao et al. Genbank acc number: AF049064; Punt et al., (1987) Gene 56 (1), 117-124), all of which can be used for expressing MSM or VMSM, preferably VMSM in yeast strains.

Examples of genetic control sequences which are suitable for expression in insect cells are the polyhedrin promoter and the p10 promoter (Luckow, V.A. and Summers, M.D. (1988) Bio/Techn. 6, 47-55).

Advantageous genetic control sequences for expressing MSM or VMSM, preferably VMSM in cell culture, in addition to polyadenylation sequences, are, for example, eukaryotic promoters from Simian virus 40 of viral origin such as, for example, promoters of the polyomavirus, adenovirus 2, cytomegalovirus, or simian virus 40.

Further advantageous genetic control sequences for expressing MSM or VMSM, preferably VMSM, in plants are present in the plant promoters CaMV/35S [Franck et al., Cell 21(1980) 285-294], PRP1 [Ward et al., Plant. Mol. Biol. 22 (1993)], SSU, OCS, LEB4, USP, STLS1, B33, NOS; FBPaseP (WO 98/18940) or in the ubiquitin or phaseolin promoter; preferably, a plant promoter or promoter derived from a plant virus is used in particular. Especially preferred are promoters of viral origin, such as the promoter of the cauliflower mosaic virus 35S transcript (Franck et al., Cell 21 (1980), 285-294; Odell et al., Nature 313 (1985), 810-812). Further preferred constitutive promoters are, for example, the promoter of the nopaline synthase from Agrobacterium, the TR dual promoter, the OCS (octopine synthase) promoter from Agrobacterium, the ubiquitin promoter (Holtorf S et al., Plant Mol Biol 1995, 29:637-649), the promoters of the vacuolar ATPase subunits or the promoter of a prolin-rich protein from wheat (WO 91/13991).

The expression cassettes may also comprise a chemically inducible promoter as genetic control sequence by means of which the expression of the exogenous gene can be controlled, in the plant, at a specific point in time. Such promoters such as, for example, the PRP1 promoter (Ward et al., Plant. Mol. Biol. 22 (1993), 361-366), a salicylic-acid-inducible promoter (WO 95/19443), a benzenesulfonamide-inducible promoter (EP-A-0388186), a tetracyclin-inducible promoter (Gatz et al., (1992) Plant J. 2, 397404), an abscisic-acid-inducible promoter (EP-A 335528) or an ethanol- or cyclohexanone-inducible promoter (WO 93/21334) may likewise be used.

Furthermore, suitable promoters are those which confer tissue- or organ-specific expression in, for example, anthers, ovaries, flowers and floral organs, leaves, stomata, trichomes, stems, vascular tissues, roots and seeds. Others which are suitable in addition to the abovementioned constitutive promoters are, in particular, those promoters which ensure leaf-specific expression. Promoters which must be mentioned are the potato cytosolic FBPase promoter (WO 97/05900), the rubisco (ribulose-1,5-bisphosphate carboxylase) SSU (small subunit) promoter or the ST-LSI promoter from potato (Stockhaus et al., EMBO J. 8 (1989), 2445-245). Promoters which are furthermore preferred are those which control expression in seeds and plant embryos. Examples of seed-specific promoters are the phaseolin promoter (US 5,504,200, Bustos MM et al., Plant Cell. 1989;1(9):839-53), the promoter of the 2S albumin gene (Joseffson LG et al., J Biol Chem 1987, 262:12196-12201), the legumin promoter (Shirsat A et al., Mol Gen Genet. 1989; 215(2):326-331), the USP (unknown seed protein) promoter (Bäumlein H et al., Molecular & General Genetics 1991, 225(3): 459-67), the napin gene promoter (Stalberg K, et al., L. Planta 1996, 199:515-519), the sucrose binding protein promoter (WO 00/26388) or the LeB4 promoter (Bäumlein H et al., Mol Gen Genet 1991, 225: 121-128; Fiedler, U. et al., Biotechnology (NY) (1995), 13 (10) 1090).

Further promoters which are suitable as genetic control sequences are, for example, specific promoters for tubers, storage roots or roots, such as, for example, the class I patatin promoter (B33), the potato cathepsin D inhibitor promoter, the starch synthase (GBSS1) promoter or the sporamin promoter, fruit-specific promoters such as, for example, the fruit-maturation-specific promoter from tomato (EP-A409625), fruit-maturation-specific promoters such as, for example, the fruit-maturation-specific promoter from tomato (WO 94/21794), inflorescence-specific promoters such as, for example, the phytoene synthase promoter (WO 92/16635) or the promoter of the P-rr gene (WO 98/22593), or plastid- or chromoplast-specific promoters such as, for example, the RNA polymerase promoter (WO 97/06250), or else the Glycine max phosphoribosyl-pyrophosphate amidotransferase promoter (see also Genbank Accession No. U87999), or another node-specific promoter as described in EP-A 249676 can advantageously be used.

Additional functional elements b) are understood as meaning, by way of example but not by limitation, reporter genes, replication origins, selection markers and what are known as affinity tags, in fusion with MSM or VMSM, preferably VMSM, directly or by means of a linker optionally comprising a protease cleavage site. Further suitable additional functional elements are sequences which ensure that the product is targeted into the apoplasts, into plastids, the vacuole, the mitochondrion, the peroxisome, the endoplasmatic reticulum (ER) or, owing to the absence of such operative sequences, remains in the compartment where it is formed, to the cytosol, (Kermode, Crit. Rev. Plant Sci. 15, 4 (1996), 285-423).

Also in accordance with the invention are vectors comprising at least one copy of the nucleic acid sequences according to the invention and/or the expression cassettes according to the invention.

In addition to plasmids, vectors are furthermore also understood as meaning all of the other known vectors with which the skilled worker is familiar, such as, for example, phages, viruses such as SV40, CMV, baculovirus, adenovirus, transposons, IS elements, phasmids, phagemids, cosmids or linear or circular DNA. These vectors can be replicated autonomously in the host organism or replicated chromosomally; chromosomal replication is preferred.

In a further embodiment of the vector, the nucleic acid construct according to the invention can advantageously also be introduced into the organisms in the form of a linear DNA and integrated into the genome of the host organism via heterologous or homologous recombination. This linear DNA may consist of a linearized plasmid or only of the nucleic acid construct as vector, or the nucleic acid sequences used.

Further prokaryotic or eukaryotic expression systems are mentioned in Chapters 16 and 17 in Sambrook et al., "Molecular Cloning: A Laboratory Manual." 2nd ed., Cold Spring Harbor Laboratory, Cold Spring Harbor Laboratory Press, Cold Spring Harbor, NY,1989. Further advantageous vectors are described in Hellens et al. (Trends in plant science, 5, 2000).

The expression cassette according to the invention and vectors derived therefrom can be used for transforming bacteria, cyanobacteria, (for example of the genus Synechocystis, Anabaena, Calothrix, Scytonema, Oscillatoria, Plectonema and Nostoc), proteobacteria such as, for example, Magnetococcus sp. MC1, yeasts, filamentous fungi and algae and eukaryotic nonhuman cells (for example insect cells) with the aim of producing MSM or VMSM, preferably VMSM, recombinantly, the generation of a suitable expression cassette depending on the organism in which the gene is to be expressed.

Vectors comprising an expression cassette which contains
a) genetic control sequences in operable linkage with a VMSM sequence;
b) additional functional elements; or
c) a combination of a) and b);
form part of the subject-matter of the present invention.

In a further advantageous embodiment, the nucleic acid sequences used in the method according to the invention may also be introduced into an organism by themselves.

If, in addition to the nucleic acid sequences, further genes are to be introduced into the organism, they can all be introduced into the organism together in a single vector, or each individual gene can be introduced into the organism in each case in one vector, it being possible to introduce the different vectors simultaneously or in succession.

In this context, the introduction, into the organisms in question (transformation), of the nucleic acid(s) according to the invention, of the expression cassette or of the vector can be effected in principle by all methods with which the skilled worker is familiar.

In the case of microorganisms, the skilled worker will find suitable methods in the textbooks by Sambrook, J. et al. (1989) "Molecular cloning: A laboratory manual", Cold Spring Harbor Laboratory Press, by F.M. Ausubel et al. (1994) "Current protocols in molecular biology", John Wiley and Sons, by D.M. Glover et al., DNA Cloning Vol.1, (1995), IRL Press (ISBN019963476-9), by Kaiser et al. (1994) Methods in Yeast Genetics, Cold Spring Harbor Laboratory Press or Guthrie et al. "Guide to Yeast Genetics and Molecular Biology", Methods in Enzymology,1994, Academic Press. In the transformation of filamentous fungi, the methods of choice are firstly the generation of protoplasts and transformation with the aid of PEG (Wiebe et al. (1997) Mycol. Res. 101 (7): 971-877; Proctor et al. (1997) Microbiol. 143, 2538-2591), and secondly the transformation with the aid of Agrobacterium tumefaciens (de Groot et al. (1998) Nat. Biotech. 16, 839-842).

In the case of dicots, the methods which have been described for the transformation and regeneration of plants from plant tissues or plant cells can be exploited for transient or stable transformation. Suitable methods are the biolistic method or the transformation of protoplasts (cf., for example, Willmitzer, L.,1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basle-Cambridge), electroporation, the incubation of dry embryos in DNA-containing solution, microinjection and the agrobacterium-radiated gene transfer. The abovementioned methods are described, for example, in B. Jenes et al., Techniques for Gene Transfer, in: Transgenic Plants, Vol. 1, Engineering and Utilization, edited by S.D. Kung and R. Wu, Academic Press (1993)-143 and in Potrykus, Annu. Rev. Plant Physiol. Plant Molec.Biol. 42 (1991) 205-225).

The transformation by means of agrobacteria, and the vectors to be used for the transformation, are known to the skilled worker and described extensively in the literature (Bevan et al., Nucl. Acids Res. 12 (1984) 8711. The intermediary vectors can be integrated into the agrobacterial Ti or Ri plasmid by means of homologous recombination owing to sequences which are homologous to sequences in the T-DNA. This plasmid additionally contains the vir region, which is required for the transfer of the T-DNA. Intermediary vectors are not capable of replication in agrobacteria. The intermediary vector can be transferred to Agrobacterium tumefaciens by means of a helper plasmid (conjugation). Binary vectors are capable of replication both in E. coli and in agrobacteria. They contain a selection marker gene and a linker or polylinker which are framed by the right and left T-DNA border region. They can be transformed directly into the agrobacteria (Holsters et al. Mol. Gen. Gene. 163 (1978), 181-187), EP A 0 120 516; Hoekema, in: The Binary Plant Vector System Offsetdrukkerij Kanters B.V., Alblasserdam (1985), Chapter V; Fraley et al., Crit. Rev. Plant. Sci., 4: 1-46 and An et al. EMBO J. 4 (1985), 277-287).

The transformation of monocots by means of vectors based on agrobacterium has also been described (Chan et al., Plant Mol. Biol. 22(1993), 491-506; Hiei et al., Plant J. 6 (1994)-282; Deng et al. Science in China 33 (1990), 28-34; Wilmink et al., Plant Cell Reports 11,(1992) 76-80; May et al. Biotechnology 13 (1995) 486-492; Conner and Domisse; Int. J. Plant Sci. 153 (1992) 550-555; Ritchie et al. Transgenic Res. (1993) 252-265). Alternative systems for the transformation of monocots are the transformation by means of the biolistic approach (Wan and Lemaux; Plant Physiol. 104 (1994), 37-48; Vasil et al. Biotechnology 11 (1992), 667-674; Ritala et al., Plant Mol. Biol 24, (1994) 317-325; Spencer et al., Theor. Appl. Genet. 79 (1990), 625-631), protoplast transformation, the electroporation of partially permeabilized cells, and the introduction of DNA by means of glass fibers. In particular the transformation of maize has been described repeatedly in the literature (cf., for example, WO 95/06128; EP 0513849 A1; EP 0465875 A1; EP 0292435 A1; Fromm et al., Biotechnology 8 (1990), 833-844; Gordon-Kamm et al., Plant Cell 2 (1990), 603-618; Koziel et al., Biotechnology 11(1993) 194-200; Moroc et al., Theor Applied Genetics 80 (190) 721-726).

The successful transformation of other cereal species has also already been described for example in the case of barley (Wan and Lemaux, see above; Ritala et al., see above; wheat (Nehra et al., Plant J. 5(1994) 285-297).

Agrobacteria which have been transformed with a vector according to the invention can likewise be used in a known manner for the transformation of plants, such as test plants like Arabidopsis or crop plants like cereals, maize, oats, rye, barley, wheat, soya, rice, cotton, sugarbeet, canola, sunflower, flax, hemp, potato, tobacco, tomato, carrot, capsicum, oilseed rape, tapioca, cassava, arrowroot, Tagetes, alfalfa, lettuce and the various tree, nut and grapevine species, for example by bathing scarified leaves or leaf segments in an agrobacterial solution and subsequently growing them in suitable media.

The genetically modified plant cells can be regenerated via all methods with which the skilled worker is familiar. Such methods can be found in the abovementioned publications by S.D. Kung and R. Wu, Potrykus or Höfgen and Willmitzer.

The transgenic organisms generated by transformation with one of the above-described embodiments of an expression cassette comprising a nucleic acid sequence according to the invention or a vector comprising the abovementioned expression cassette, and the recombinant VMSM, which can be obtained from the transgenic organism by means of expression, form part of the subject matter of the present invention. The use of transgenic organisms comprising an expression cassette according to the invention, for example for providing recombinant protein, and/or the use of these organisms in in-vivo assay systems likewise form part of the subject matter of the present invention.

Preferred organisms for the recombinant expression are not only bacteria, yeasts, mosses, algae and fungi, but also eukaryotic cell lines.

Preferred mosses are Physcomitrella patens or other mosses described in Kryptogamen [Cryptogamia], Vol.2, Moose, Farne [Mosses, Ferns],1991, Springer Verlag (ISBN 3540536515).

Preferred within the bacteria are, for example, bacteria from the genus Escherichia, Erwinia, Flavobacterium, Alcaligenes or cyanobacteria, for example from the genus Synechocystis, Anabaena, Calothrix, Scytonema, Oscillatoria, Plectonema and Nostoc, especially preferably Synechocystis or Anabaena.

Preferred yeasts are Candida, Saccharomyces, Schizosaccheromyces, Hansenula or Pichia.

Preferred fungi are Aspergillus, Trichoderma, Ashbya, Neurospora, Fusarium, Beauveria, Mortierella, Saprolegnia, Pythium, or other fungi described in Indian Chem Engr. Section B. Vol 37, No 1,2 (1995).

Preferred plants are selected in particular among monocotyledonous crop plants such as, for example, cereal species such as wheat, barley, millet, rye, triticale, maize, rice or oats, and sugarcane. The transgenic plants according to the invention are, furthermore, in particular selected from among dicotyledonous crop plants such as, for example, Brassicacae such as oilseed rape, cress, Arabidopsis, cabbages or canola; Leguminosae such as soyabean, alfalfa, pea, beans or peanut, Solanaceae such as potato, tobacco, tomato, egg plant or capsicum; Asteraceae such as sunflower, Tagetes, lettuce or Calendula; Cucurbitaceae such as melon, pumpkin or zucchini, or linseed, cotton, hemp, flax, red pepper, carrot, sugar beet, or various tree, nut and grapevine species.

In principle, transgenic animals such as, for example, C. elegans, are also suitable as host organisms.

Also preferred is the use of expression systems and vectors which are available to the public or commercially available.

Those which must be mentioned for use in E. coli bacteria are the typical advantageous commercially available fusion and expression vectors pGEX [Pharmacia Biotech Inc; Smith, D.B. and Johnson, K.S. (1988) Gene 67:31-40], pMAL (New England Biolabs, Beverly, MA) and pRIT5 (Pharmacia, Piscataway, NJ), which contains glutathione S transferase (GST), maltose binding protein or protein A, the pTrc vectors (Amann et al., (1988) Gene 69:301-315), "pKK233-2" from CLONTECH, Palo Alto, CA and the "pET", and the "pBAD" vector series from Stratagene, La Jolla.

Further advantageous vectors for use in yeast are pYepSecl (Baldari, et al., (1987) Embo J. 6:229-234), pMFa (Kurjan and Herskowitz, (1982) Cell 30:933-943), pJRY88 (Schultz et al., (1987) Gene 54:113-123), and pYES derivatives, pGAPZ derivatives, pPICZ derivatives, and the vectors of the "Pichia Expression Kit" (Invitrogen Corporation, San Diego, CA). Vectors for use in filamentous fungi are described in: van den Handel, C.A.M.J.J. & Punt, P.J. (1991) "Gene transfer systems and vector development for filamentous fungi, in: Applied Molecular Genetics of Fungi, J.F. Peberdy, et al., eds., p. 1-28, Cambridge University Press: Cambridge.

As an alternative, insect cell expression vectors may also be used advantageously, for example for expression in Sf9, Sf21 or Hi5 cells, which are infected via recombinant Baculoviruses. Examples of these are the vectors of the pAc series (Smith et al. (1983) Mol. Cell Biol. 3:2156-2165) and the pVL series (Lucklow and Summers (1989) Virology 170:31-39). Others which may be mentioned are the Baculovirus expression systems "MaxBac 2.0 Kit" and "Insect Select System" from Invitrogen, Calsbad or "BacPAK Baculovirus Expression System" from CLONTECH, Palo Alto, CA. Insect cells are particularly suitable for overexpressing eukaryotic proteins since they effect posttranslational modifications of the proteins which are not possible in bacteria and yeasts. The skilled worker is familiar with the handling of cultured insect cells and with their infection for expressing proteins, which can be carried out analogously to known methods (Luckow and Summers, Bio/Tech. 6,1998, pp.47-55; Glover and Hames (eds) in DNA Cloning 2, A practical Approach, Expression Systems, Second Edition, Oxford University Press,1995, 205-244).

Plant cells or algal cells are others which can be used advantageously for expressing genes. Examples of plant expression vectors can be found as mentioned above in Becker, D., et al. (1992) "New plant binary vectors with selectable markers located proximal to the left border", Plant Mol. Biol. 20: 1195-1197 or in Bevan, M.W. (1984) "Binary Agrobacterium vectors for plant transformation", Nucl. Acid. Res. 12: 8711-8721.

Moreover, the nucleic acid sequences according to the invention can be expressed in mammalian cells. Examples of suitable expression vectors are pCDM8 and pMT2PC, which are mentioned in: Seed, B. (1987) Nature 329:840 or Kaufman et al. (1987) EMBO J. 6:187-195). Promoters preferably to be used in this context are of viral origin such as, for example, promoters of polyomavirus, adenovirus 2, cytomegalovirus or simian virus 40. Further prokaryotic and eukaryotic expression systems are mentioned in Chapter 16 and 17 in Sambrook et al., Molecular Cloning: A Laboratory Manual. 2nd ed., Cold Spring Harbor Laboratory, Cold Spring Harbor Laboratory Press, Cold Spring Harbor, NY, 1989. Further advantageous vectors are described in Hellens et al. (Trends in plant science, 5, 2000).

The transgenic organisms which comprise VMSM sequences are claimed within the scope of the present invention.

All of the above-described embodiments of the transgenic organisms which comprise MSM or VMSM, preferably VMSM, come under the term "transgenic organism according to the invention".

The invention furthermore relates to the use of MSM or VMSM, preferably VMSM, in a method for identifying herbicidally active test compounds.

The method according to the invention for identifying herbicidally active compounds preferably comprises the following steps:
i. bringing MSM or VMSM, preferably VMSM, into contact with one or more test compounds under conditions which permit binding of the test compound(s) to MSM or VMSM, preferably VMSM; and
ii. detecting whether the test compound binds to MSM or VMSM, preferably the VMSM of i); or
iii. detecting whether the test compound reduces or blocks the activity of MSM or VMSM, preferably the VMSM of i); or
iv. detecting whether the test compound reduces or blocks the transcription, translation or expression of a MSM or VMSM, preferably the VMSM of i).

The term "reduced" is understood as meaning reduction of the activity of at least 10%, advantageously at least 20%, preferably at least 50%, especially preferably by at least 70% and very especially preferably by at least 80%, 90% or 95% in comparison with the activity of the MSM or VMSM, preferably VMSM, which has not been brought into contact with a test compound; the term "blocked" is understood as meaning the complete, i.e. 100%, blocking of the activity, where the abovementioned reductions in percent are achieved at an inhibitor concentration of less than 10⁻⁴M, preferably less than 10⁻⁵M, especially preferably less than 10⁻⁶M and very especially preferably less than 10⁻⁷M.

The detection in accordance with step (ii) of the above method can be effected using techniques which identify the interaction between protein and ligand. In this context, either the test compound or the enzyme can contain a detectable label such as, for example, a fluorescent label, a radioisotope label, a chemiluminescent label or an enzyme label. Examples of enzyme labels are horseradish peroxidase, alkaline phosphatase or luciferase. The subsequent detection depends on the label and is known to the skilled worker.

In this context, five preferred embodiments which are also suitable for high-throughput methods (High Throughput Screening HTS) in connection with the present invention must be mentioned in particular:
1. The average diffusion rate of a fluorescent molecule as a function of the mass can be determined in a small sample volume via fluorescence correlation spectroscopy (FCS) (Proc. Natl. Acad. Sci. USA (1994) 11753-11575). FCS can be employed for determining protein/ligand interactions by measuring the change in the mass, or the changed diffusion rate which this entails, of a test compound when binding to MSM or VMSM, preferably VMSM. A method according to the invention can be designed directly for measuring the binding of a test compound labeled by a fluorescent molecule. As an alternative, the method according to the invention can be designed in such a way that a chemical reference compound which is labeled by a fluorescent molecule is displaced by further test compounds ("displacement assay").
2. Fluoresence polarization exploits the characteristic of a quiescent fluorophore excited with polarized light to likewise emit polarized light. If, however, the fluorophore is allowed to rotate during the excited state, the polarization of the fluorescent light which is emitted is more or less lost. Under otherwise identical conditions (for example temperature, viscosity, solvent), the rotation is a function of molecule size, whereby findings regarding the size of the fluorophore-bound residue can be obtained via the reading (Methods in Enzymology 246 (1995), pp. 283-300). A method according to the invention can be designed directly for measuring the binding of a test compound labeled with a fluorescent molecule to MSM or VMSM, preferably VMSM. As an alternative, the method according to the invention may also take the form of the "displacement assay" described under 1.
3. Fluorescence resonance energy transfer (FRET) is based on the irradiation-free energy transfer between two spatially adjacent fluorescent molecules under suitable conditions. A prerequisite is that the emission spectrum of the donor molecule overlaps with the excitation spectrum of the acceptor molecule. The fluorescent label of MSM or VMSM, preferably VMSM, and binding test compound, the binding can be measured by means of FRET (Cytometry 34, 1998, pp. 159-179). As an alternative, the method according to the invention may also take the form of the "displacement assay" described under 1. An especially suitable embodiment of FRET technology is "Homogeneous Time Resolved Fluorescence" (HTRF) as can be obtained from Packard BioScience.
4. Surface-enhanced laser desorption/ionization (SELDI) in combination with a time-of-flight mass spectrometer (MALDI-TOF) makes possible the rapid analysis of molecules on a support and can be used for analyzing protein/ligand interactions (Worral et al., (1998) Anal. Biochem. 70:750-756). In a preferred embodiment, MSM or VMSM, preferably VMSM, is immobilized on a suitable support and incubated with the test compound. After one or more suitable wash steps, the test compound molecules which are additionally bound to MSM or VMSM, preferably VMSM, can be detected by means of the abovementioned methodology and test compounds which are bound to MSM or VMSM, preferably VMSM, can thus be selected.
5. The measurement of surface plasmon resonance is based on the change in the refractive index at a surface when a test compound binds to a protein which is immobilized to said surface. Since the change in the refractive index is identical for virtually all proteins and polypeptides for a defined change in the mass concentration at the surface, this method can be applied to any protein in principle (Lindberg et al. Sensor Actuators 4 (1983) 299-304; Malmquist Nature 361 (1993) 186-187). The measurement can be carried out for example with the automatic analyzer based on surface plasmon resonance which is available from Biacore (Freiburg) at a throughput of, currently, up to 384 samples per day. A method according to the invention can be designed directly for measuring the binding of a test compound to MSM or VMSM, preferably VMSM. As an alternative, the method according to the invention may also take the form of the "displacement assay" described under 1.

The compounds identified via the abovementioned methods 1 to 5 may be suitable as inhibitors. All of the substances identified via the abovementioned methods can subsequently be checked for their herbicidal action in another embodiment of the method according to the invention.

Furthermore, there exists the possibility of detecting further candidates for herbicidal active ingredients by molecular modeling via elucidation of the three-dimensional structure of MSM or VMSM, preferably VMSM, by x-ray structure analysis. The preparation of protein crystals required for x-ray structure analysis, and the relevant measurements and subsequent evaluations of these measurements, the detection of a binding site in the protein, and the prediction of potential inhibitor structures are known to the skilled worker. In principle, an optimization of the compounds identified by the abovementioned methods is also possible via molecular modeling.

A preferred embodiment of the method according to the invention, which is based on steps i) and ii), consists in selecting a test compound which reduces or blocks the enzymatic activity of the MSM or VMSM, preferably VMSM, the activity of the MSM or VMSM, preferably VMSM, incubated with the test compound being compared with the activity of a MSM or VMSM, preferably VMSM, not incubated with a test compound.

A preferred embodiment of the method based on steps i) and ii) consists in
i. expressing a MSM or VMSM, preferably VMSM, in a transgenic organism according to the invention or growing an organism which naturally contains a MSM or VMSM, preferably VMSM,
ii. bringing the MSM or VMSM, preferably VMSM, of step i) in the cell digest of the transgenic or nontransgenic organism, in partially purified or in homogeneously purified form, into contact with a test compound; and
iii. selecting a compound which reduces or blocks the activity of the MSM or VMSM, preferably the VMSM.

In step iii. here to determine the activity of the MSM or VMSM, preferably the VMSM, incubated with the test compound can be compared with the activity of an MSM or VMSM, preferably VMSM, not incubated with a test compound.

The solution comprising MSM or VMSM, preferably VMSM, can consist of the lyzate of the original organism. As an alternative, the solution comprising the MSM or VMSM, preferably VMSM, can consist of the lyzate of the transgenic organism which has been transformed with an expression cassette according to the invention.

MSM or VMSM, preferably VMSM, can be partly or fully purified via customary methods, if so required. A general review over customary protein purification techniques is given for example in Ausubel, F.M. et al., Current Protocols in Molecular Biology, Greene Publishing Assoc. and Wiley-Interscience (1994); ISBN 0-87969-309-6. In the case of recombinant production, purification of the protein, which is fused to an affinity tag, can be affected via affinity chromatography, with which the skilled worker is familiar.

The MSM or VMSM, preferably VMSM, required for in-vitro methods can thus be isolated either by means of heterologous expression from a transgenic organism according to the invention or from an organism comprising MSM or VMSM, preferably VMSM, for example from plant chloroplasts.

To identify herbicidal compounds, the MSM or VMSM, preferably VMSM, is now incubated with a test compound. After a reaction time, the enzymatic activity of the MSM or VMSM, preferably VMSM, incubated with the test compound is determined in comparison with an MSM or VMSM, preferably VMSM, not incubated with a test compound. If the MSM or VMSM, preferably VMSM, is inhibited, a significant decrease in activity in comparison with the activity of the noninhibited polypeptide according to the invention is observed, the result being a reduction of at least 10%, advantageously at least 20%, preferably at least 30%, especially preferably by at least 50%, up to 100% reduction (blocking). Preferred is an inhibition of at least 50% at test compound concentrations of 10⁻⁴M, preferably at 10⁻⁵M, especially preferably of 10⁻⁶M, very especially preferably of 10⁻⁷M based on enzyme concentration in the micromolar range.

The activity of MSM or VMSM, preferably VMSM, can be determined for example by an activity assay in which the increase of the product, the decrease of the substrate (or starting material) or the decrease or increase of the cofactor are determined, or by a combination of at least two of the abovementioned parameters, as a function of a defined period of time.

The substrate quantities to be employed in the activity assay can amount to between 0.1-10 mM and the cofactor quantities to between 0.1-10 mM based on 1-100 µg/ml MSM or VMSM enzyme.

Examples of suitable substrates for determining the activity of MSM or VMSM are, for example, 2-methyl-6-solanylbenzoquinol, 2-methyl-6-phytylbenzoquinol, 2-methyl-6-geranylgeranylbenzoquinol and 2-methylbenzoquinol derivatives with a shortened prenyl chain in the 6-position.

An example of a suitable cofactor is S-adenosylmethionine.

If appropriate, derivatives of the abovementioned compounds which contain a detectable label such as, for example, a fluorescent label, a radioisotope label (for example [¹⁴C-methyl]S-adenosylmethionine) or a chemiluminescent label, may also be used.

Thus, the activity of MSM or VMSM in step iii) of the abovementioned method can be determined photometrically by the method of Peddibhotla et al. (2003 Tetrahedron Letters 44, pp. 237-239).

An alternative is the detection of the reaction products after separation via HPLC. The reaction products can be detected by means of radioactive photometric or electrochemical detection.

Another possibility is photometric monitoring of the reaction based on the characteristic chromophoric properties of the quinoid substrates and products.

A preferred embodiment of the method according to the invention which is based on steps i) and iii) consists of the following steps:
i. generation of a transgenic organism comprising at least one nucleic acid sequence encoding MSM or VMSM, preferably VMSM, in which MSM or VMSM, preferably VMSM is overexpressed,
ii. applying a test compound to the transgenic organism of i) and to a nontransgenic organism of the same genotype;
iii. determining the growth or the viability of the transgenic and the nontransgenic organisms after application of the test substance; and
iv. selecting test compounds which bring about a reduced growth or a limited viability of the nontransgenic organism in comparison with the growth of the transgenic organism.

In this context, the difference in growth in step iv) for the selection of a herbicidally active inhibitor amounts to at least more than 10%, by preference more than 20%, preferably more than 30%, especially preferably more than 40% and very especially preferably more than 50%.

The term "transgenic organism" is understood as meaning the abovementioned transgenic organisms according to the invention.

As an alternative, a transgenic organism in which MSM or VMSM, preferably VMSM, is overexpressed and which is suitable for the abovementioned method can also be generated by bringing about the overexpression of MSM or VMSM, preferably VMSM, by manipulation of the promoter sequences which are naturally present in the organism. The skilled worker is familiar with such methods.

The transgenic organism in this context is preferably a plant, an alga, a cyanobacterium, for example of the genus Synechocystis or a proteobacterium such as, for example, Magnetococcus sp. MC1, preferably plants which can be transformed by means of customary techniques, such as Arabidopsis thaliana, Solanum tuberosum, Nicotiana Tabacum, or cyanobacteria which can be transformed readily, such as Synechocystis, into which the sequence encoding a polypeptide according to the invention has been incorporated by transformation. These transgenic organisms thus show increased tolerance to compounds which inhibit the polypeptide according to the invention. "Knock-out" mutants in which the analogous MSM or VMSM, preferably VMSM, gene(s) which is/are naturally present in this organism has/have been selectively switched off may also be used.

However, the abovementioned embodiment of the method according to the invention can also be used for identifying substances with a growth-regulatory action. In this context, the transgenic organism employed is a plant. The method for identifying substances with growth-regulatory activity thus comprises the following steps:
i. generating a transgenic plant comprising a nucleic acid sequence encoding MSM or VMSM, preferably VMSM, which is overexpressed
ii. applying a test substance to the transgenic plant of i) and to a nontransgenic plant of the same variety,
iii. determining the growth or the viability of the transgenic plant and the nontransgenic plant after application of the test compound; and
iv. selecting test substances which bring about a modified growth of the nontransgenic plant in comparison with the growth of the transgenic plant.

Here, step iv) involves the selection of test compounds which bring about a modified growth of the nontransgenic organism in comparison with the growth of the transgenic organism. Modified growth is understood as meaning, in this context, inhibition of the vegetative growth of the plants, which can manifest itself in particular in reduced longitudinal growth. Accordingly, the treated plants show stunted growth; moreover, their leaves are darker in color. In addition, modified growth is also understood as meaning a change in the course of maturation over time, an inhibition or promotion of lateral branched growth of the plants, shortened or extended developmental stages, increased standing ability, the growth of larger amounts of buds, flowers, leaves, fruits, seed kernels, roots and tubers, an increased sugar content in plants such as sugarbeet, sugar cane and citrus fruit, an increased protein content in plants such as cereals or soybean, or stimulation of the latex flow in rubber trees. The skilled worker is familiar with the detection of such modified growth.

Again, as an alternative, the transgenic plant in which MSM or VMSM, preferably VMSM, can be generated by bringing about the overexpression of MSM or VMSM, preferably VMSM, by manipulating the promoter sequences which are naturally present in the organism. The skilled worker is familiar with such methods.

It is also possible, in the method according to the invention, to employ a plurality of test compounds in a method according to the invention. If a group of test compounds affect the target, then it is either possible directly to isolate the individual test compounds or to divide the group of test compounds into a variety of subgroups, for example when it consists of a multiplicity of different components, in order to thus reduce the number of the different test compounds in the method according to the invention. The method according to the invention is then repeated with the individual test compound or the relevant subgroup of test compounds. Depending on the complexity of the sample, the above-described steps can be carried out repeatedly, preferably until the subgroup identified in accordance with the method according to the invention only comprises a small number of test compounds, or indeed just one test compound.

All of the above-described methods for identifying inhibitors with herbicidal or growth-regulatory activity are hereinbelow referred to as "methods according to the invention". Here, "method according to the invention" preferably refers to the above-described methods for identifying herbicidally active inhibitors.

All of the compounds which have been identified via the methods according to the invention can subsequently be tested in vivo for their herbicidal and growth-regulatory activity. One possibility of testing the compounds for herbicidal action is to use duckweed, Lemna minor, in microtiter plates. Parameters which can be measured are changes in the chlorophyll content and the photosynthesis rate. It is also possible to apply the compound directly to undesired plants, it being possible to identify the herbicidal action for example via restricted growth.

The method according to the invention can advantageously also be carried out in high-throughput methods, known as HTS, which makes possible the simultaneous testing of a multiplicity of different compounds.

The use of supports which contain one or more of the nucleic acid molecules according to the invention, one or more of the vectors containing the nucleic acid sequence according to the invention, one or more transgenic organisms containing at least one of the nucleic acid sequences according to the invention or one or more (poly)peptides encoded via the nucleic acid sequences according to the invention lends itself to carrying out HTS in practice. The support used can be solid or liquid, but is preferably solid and especially preferably a microtiter plate. The abovementioned supports also form part of the subject matter of the present invention. In accordance with the most widely used technique, 96-well, 384-well and 1536-well microtiter plates which, as a rule, can comprise volumes of up to 200 µl, are used. Besides the microtiter plates, the further components of an HTS system which match the corresponding microtiter plates, such as a large number of instruments, materials, automatic pipetting devices, robots, automated plate readers and plate washers, are commercially available.

In addition to the HTS systems based on microtiter plates, what are known as "free-format assays" or assay systems where no physical barriers exist between the samples, as described, for example, in Jayaickreme et al., Proc. Natl. Acad. Sci U.S.A. 19 (1994) 161418; Chelsky, "Strategies for Screening Combinatorial Libraries", First Annual Conference of The Society for Biomolecular Screening in Philadelphia, Pa. (Nov. 710, 1995); Salmon et al., Molecular Diversity 2 (1996), 5763 and US 5,976,813, may also be used.

The invention furthermore relates to herbicidally active compounds identified by the methods according to the invention. These compounds are hereinbelow referred to as "selected compounds". They have a molecular weight of less than 1000 g/mol, advantageously less than 500 g/mol, preferably less than 400 g/mol, especially preferably less than 300 g/mol. Herbicidally active compounds have a Ki value of less than 1 mM, preferably less than 1 µM, especially preferably less than 0.1 µM, very especially preferably less than 0.01 µM.

The invention furthermore relates to compounds with growth-regulatory activity identified by the methods according to the invention. These compounds too are hereinbelow referred to as "selected compounds". However, the term "selected compounds" preferably represents herbicidally active compounds.

Naturally, the selected compounds can also be present in the form of their agriculturally useful salts. Agriculturally useful salts which are suitable are mainly the salts of those cations, or the acid addition salts of those acids, whose cations, or anions, do not adversely affect the herbicidal action of the herbicidally active compounds identified via the methods according to the invention.

If the selected compounds contain asymmetrically substituted α-carbon atoms, they may furthermore also be present in the form of racemates, enantiomer mixtures, pure enantiomers or, if they have chiral substituents, also in the form of diastereomer mixtures.

The selected compounds can be chemically synthesized substances or substances produced by microbes and can be found, for example, in cell extracts of, for example, plants, animals or microorganisms. The reaction mixture can be a cell-free extract or comprise a cell or cell culture. Suitable methods are known to the skilled worker and are described generally for example in Alberts, Molecular Biology the cell, 3rd Edition (1994), for example chapter 17. The selected compounds may also originate from comprehensive substance libraries.

Candidate test compounds can be expression libraries such as, for example, cDNA expression libraries, peptides, proteins, nucleic acids, antibodies, small organic substances, hormones, PNAs or the like (Milner, Nature Medicin 1 (1995), 879-880; Hupp, Cell. 83 (1995), 237-245; Gibbs, Cell. 79 (1994), 193-198 and references cited therein).

The selected compounds can be used for controlling undesired vegetation and/or as growth regulators. Herbicidal compositions comprising the selected compounds afford very good control of vegetation on noncrop areas. In crops such as wheat, rice, maize, soybean and cotton, they act against broad-leaved weeds and grass weeds without inflicting any significant damage on the crop plants. This effect is observed in particular at low application rates. The selected compounds can be used for controlling the harmful plants which have already been mentioned above.

Depending on the application method in question, selected compounds, or herbicidal compositions comprising them, can advantageously also be employed in a further number of crop plants for eliminating undesired plants. Examples of suitable crops are:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

In addition, the selected compounds can also be used in crops which tolerate the action of herbicides owing to breeding, including recombinant methods. The generation of such crops is described hereinbelow.

The invention furthermore relates to a method of preparing the herbicidal or growth-regulatory composition which has already been mentioned above, which comprises formulating selected compounds with suitable auxiliaries to give crop protection products.

The selected compounds can be formulated for example in the form of directly sprayable aqueous solutions, powders, suspensions, also highly concentrated aqueous, oily or other suspensions or suspoemulsions or dispersions, emulsifiable concentrates, emulsions, oil dispersions, pastes, dusts, materials for spreading or granules, and applied by means of spraying, atomizing, dusting, spreading or pouring. The use forms depend on the intended use and the nature of the selected compounds; in any case, they should guarantee the finest possible distribution of the selected compounds. The herbicidal compositions comprise a herbicidally active amount of at least one selected compound and auxiliaries conventionally used in the formulation of herbicidal compositions.

For the preparation of emulsions, pastes or aqueous or oily formulations and dispersible concentrates (DC), the selected compounds can be dissolved or dispersed in an oil or solvent, it being possible to add further formulation auxiliaries for homogenization. However, it is also possible to prepare liquid or solid concentrates from the selected compound, if appropriate solvents or oil and, optionally, further auxiliaries comprising liquid or solid concentrates, and these concentrates are suitable for dilution with water. The following can be mentioned: emulsifiable concentrates (EC, EW), suspensions (SC), soluble concentrates (SL), dispersible concentrates (DC), pastes, pills, wettable powders or granules, it being possible for the solid formulations either to be soluble or dispersible (wettable) in water. In addition, suitable powders or granules or tablets can additionally be provided with a solid coating which prevents abrasion or premature release of the active ingredient.

In principle, the term "auxiliaries" is understood as meaning the following classes of compounds: antifoams, thickeners, wetting agents, tackifiers, dispersants, emulsifiers, bactericides and/or thixotropic agents. The skilled worker is familiar with the meaning of the abovementioned agents.

SLs, EWs and ECs can be prepared by simply mixing the ingredients in question; powders can be prepared by mixing or grinding in specific types of mills (for example hammer mills). DCs, SCs and SEs are usually prepared by wet milling, it being possible to prepare an SE from an SC by addition of an organic phase which may comprise further auxiliaries or selected compounds. The preparation is known. Powders, materials for spreading and dusts can advantageously be prepared by mixing or cogrinding the active substances together with a solid carrier. Granules, for example coated granules, impregnated granules and homogeneous granules, can be prepared by binding the selected compounds to solid carriers. The skilled worker is familiar with further details regarding their preparation, which are mentioned for example in the following publications: US 3,060,084, EP-A 707445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York,1961, Hance et al., Weed Control Handbook, 8th Ed., Blackwell Scientific Publications, Oxford, 1989 and Mollet, H., Grubemann, A., Formulation technology, Wiley VCH Verlag GmbH, Weinheim (Federal Republic of Germany), 2001.

The skilled worker is familiar with a multiplicity of inert liquid and/or solid carriers which are suitable for the formulations according to the invention, such as, for example, liquid additives such as mineral oil fractions of medium to high boiling point such as kerosene or diesel oil, furthermore coal tar oils and oils of vegetable or animal origin, aliphatic, cyclic and aromatic hydrocarbons, for example paraffin, tetrahydrophthalene, alkylated naphthalenes or their derivatives, alkylated benzenes or their derivatives, alcohols such as methanol, ethanol, propanol, butanol and cyclohexanol, ketones such as cyclohexanone, or strongly polar solvents, for example amines such as N-methylpyrrolidone or water.

Examples of solid carriers are mineral earths such as silicas, silica gels, silicates, talc, kaolin, limestone, lime, chalk, bole, loess, clay, dolomite, diatomaceous earth, calcium sulfate, magnesium sulfate, magnesium oxide, ground synthetic materials, fertilizers such as ammonium sulfate, ammonium phosphate, ammonium nitrate, ureas and products of vegetable origin such as cereal meal, tree bark meal, wood meal and nutshell meal, cellulose powders or other solid carriers.

The skilled worker is familiar with the multiplicity of surface-active substances (surfactants) which are suitable for the formulations according to the invention such as, for example, alkali metal salts, alkaline earth metal salts or ammonium salts of aromatic sulfonic acids for example lignosulfonic acid, phenolsulfonic acid, naphthalenesulfonic acid, and dibutylnaphthalenesulfonic acid, and of fatty acids, of alkyl- and alkylarylsulfonates, of alkyl sulfates, lauryl ether sulfates and fatty alcohol sulfates, and salts of sulfated hexa-, hepta- and octadecanols and of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ethers, ethoxylated isooctyl-, octyl- or nonylphenol, alkylphenyl polyglycol ethers, tributylphenyl polyglycol ethers, alkylaryl polyether alcohols, isotridecyl alcohol, fatty alcohol/ethylene oxide condensates, ethoxylated caster oil, polyoxyethylene alkyl ethers or polyoxypropylene alkyl ethers, lauryl alcohol polyglycol ether acetate, sorbitol esters, lignosulfite waste liquors or methylcellulose.

The herbicidal compositions, or the selected compounds, can be applied pre- or post-emergence. If the selected compounds are less well tolerated by certain crop plants, application techniques may be used in which the selected compounds are sprayed, with the aid of the spraying apparatus, in such a way that they come into as little contact, if any, with the leaves of the sensitive crop plants while the selected compounds reach the leaves of undesired plants which grow underneath, or the bare soil surface (post-directed, lay-by).

Depending on the intended purpose of the control measures, the season, the target plants and the growth stage, the application rates of selected compounds amount to 0.001 to 3.0, preferably 0.01 to 1.0 kg/ha.

The provision of the herbicidal target furthermore makes possible a method for identifying an MSM or VMSM which is not, or only to a limited extent, inhibited by a herbicide which has MSM as its site of action, for example the herbicidally active selected compounds. Hereinbelow, a protein which thus differs from MSM or VMSM, preferably VMSM, is referred to as MSM variant, which is preferably encoded by a nucleic acid sequence which comprises
i) a functional equivalent of the nucleic acid sequence SEQ ID NO:3 which can be derived by back translating an amino acid sequence with at least 59% identity with SEQ ID NO:4; or
ii) a functional equivalent of the nucleic acid sequence SEQ ID NO:3 which can be derived by back translating an amino acid sequence with at least 59% identity with SEQ ID NO:4 and which is truncated by at least 20 amino acids at the C terminus and by at least 20 amino acids at the N terminus.

Functional equivalents of SEQ ID NO:3 are encoded by an amino acid sequence with at least 59%, 60%, 61%, 62%, 63%, 64%, 65% or 66%, preferably at least 67%, 68%, 69%, 70%, 71%, 72% or 73%, preferably at least 74%, 75%, 76%, 77%, 78%, 79% or 80%, preferably at least 81%, 82%, 83%, 84%, 85%, 86%, 87%, 88%, 89%, 90%, 91%, 92% or 93%, especially preferably at least 94%, 95%, 96%, 97%, 98% or 99% identity with SEQ ID NO:4.

All of the abovementioned nucleic acid sequences are preferably derived from a plant.

In a preferred embodiment, the abovementioned method for generating nucleic acid sequences encoding MSM variants of nucleic acid sequences comprise the following steps:
a) expression, in a heterologous system or in a cell-free system, of the proteins encoded by the abovementioned nucleic acids;
b) randomized or site-directed mutagenesis of the protein by modification of the nucleic acid;
c) measuring the interaction of the modified gene product of the herbicide;
d) identification of derivatives of the protein which exhibit a lesser degree of interaction;
e) testing the biological activity of the protein after application of the herbicide;
f) selection of the nucleic acid sequences which exhibit a modified biological activity toward the herbicide.

The sequences selected by the above-described method are advantageously introduced into an organism. The invention therefore furthermore relates to an organism prepared by this method. The organism is preferably a plant, preferably one of the above-defined crop plants.

Thereafter, intact plants are regenerated, and the resistance toward the selected compound is verified in intact plants.

Modified proteins and/or nucleic acids which are capable of conferring, in plants, resistance to the selected compounds can also be prepared from the abovementioned nucleic acid sequences via what is known as site-directed mutagenesis; by means of this mutagenesis, for example the stability and/or activity of the target protein or the characteristics such as binding of the abovementioned inhibitors according to the invention can be improved or modified in a highly specific manner.

For example, Zhu et al. (Nature Biotech., Vol. 18, May 2000: 555-558) have described a site-directed mutagenesis method in plants which can be used advantageously.

Moreover, modifications can be achieved via the PCR method described by Spee et al. (Nucleic Acids Research, Vol. 21, No. 3, 1993: 777-78) using dlTP for the random mutagenesis, or by the further-improved method of Rellos et al. (Protein Expr. Purif., 5, 1994: 270-277).

A further possibility of producing these modified proteins and/or nucleic acids is an in vitro recombination technique for molecular evolution which has been described by Stemmer et al. (Proc. Natl. Acad. Sci. USA, Vol. 91, 1994: 10747-10751), or the combination of the PCR and recombination method described by Moore et al. (Nature Biotechnology Vol. 14, 1996: 458-467).

A further way for the mutagenesis of proteins is described by Greener et al. in Methods in Molecular Biology (Vol. 57, 1996: 375-385). A method for modifying proteins using the microorganism E. coli XL-1 Red is described in EP-A-0 909 821. During its replication, this microorganism generates mutations in the nucleic acids which have been introduced and thus leads to a modification of the genetic information. Advantageous nucleic acids and the proteins encoded by them can be identified readily by isolating the modified nucleic acids or the modified proteins and carrying out resistance tests. The former can then be introduced into plants, where they are capable of manifesting the resistance and thus lead to resistance to the herbicides.

Further methods of mutagenesis and selection are, for example, methods such as the in vivo mutagenesis of seeds or pollen and the selection of resistant alleles in the presence of the inhibitors according to the invention, followed by genetic and molecular identification of the modified, resistant allele and also the mutagenesis and selection of resistances in cell culture by propagating the culture in the presence of successively increasing concentrations of the inhibitors according to the invention. In this context, the increase of the spontaneous mutation rate by chemical/physical mutagenic treatment may be exploited. As described above, modified genes can also be isolated using microorganisms which have an endogenous or recombinant activity of the proteins encoded by the nucleic acids used in the method according to the invention, and which are sensitive to the inhibitors identified in accordance with the invention. Growing the microorganisms on media with increasing concentrations of inhibitors according to the invention permits the selection and evolution of resistant variants of the targets according to the invention. The mutation frequency, in turn, can be increased by mutagenic treatments.

Methods for the specific modification of nucleic acids are furthermore available (Zhu et al. Proc. Natl. Acad. Sci. USA, Vol. 96, 8768-8773 and Beethem et al., Proc. Natl. Acad. Sci. USA, Vol. 96, 8774-8778). These methods make possible the replacement, in the proteins, of those amino acids which are important for the binding of inhibitors by functionally analogous amino acids which, however, prevent the inhibitor from binding.

The invention furthermore therefore relates to a method for establishing nucleic acid sequences which encode gene products which have a modified biological activity, the biological activity having been modified in such a way that an increased activity is present. An increased activity is understood as meaning an activity which is increased by at least 10%, preferably by at least 30%, especially preferably by at least 50%, very especially preferably by at least 100% in comparison with the starting organism, or the starting gene product. Moreover, the biological activity can have been modified in such a way that the substances and/or compositions according to the invention no longer bind, or no longer properly bind, to the nucleic acid sequences and/or the gene products encoded by them. For the purposes of the invention, no longer, or no longer properly, is to be understood as meaning that the substances bind by at least 30% less, preferably at least 50% less, especially preferably by at least 70% less, very especially preferably by at least 80% less, or indeed no longer, to the modified nucleic acids and/or gene products in comparison with the starting gene product or the starting nucleic acids.

Yet a further aspect of the invention therefore relates to a transgenic plant which has been transformed with a nucleic acid sequence which encodes a gene product with a modified biological activity, or with a nucleic acid sequence encoding an MSM or VMSM variant. Transformation methods are known to the skilled worker and detailed further above by way of example.

Genetically modified transgenic plants which are resistant to substances found in accordance with the methods according to the invention and/or to compositions comprising these substances can also be generated by transformation, followed by overexpression of a nucleic acid sequence according to the invention. The invention therefore furthermore relates to a method for the generation of transgenic plants which are resistant to substances found by a method according to the invention, which comprises overexpressing, in these plants, nucleic acids according to the invention and encoding an MSM or VMSM. A similar method is described by way of example in Lermantova et al., Plant Physiol., 122, 2000: 75 - 83.

The above-described methods according to the invention for generating resistant plants make possible the development of novel herbicides which have as complete as possible an action which is independent of the plant species (what are known as nonselective herbicides), in combination with the development of useful plants which are resistant to the nonselective herbicide. Useful plants which are resistant to nonselective herbicides have already been described on several occasions. In this context, one can distinguish between several principles for achieving a resistance:
a) Generation of resistance in a plant via mutation methods or recombinant methods by markedly overproducing the protein which acts as target for the herbicide and by the fact that, owing to the large excess of the protein which acts as target for the herbicide, the function exerted by this protein in the cell is even retained after application of the herbicide.
b) Modification of the plant such that a modified version of the protein which acts as target of the herbicide is introduced and that the function of the newly introduced modified protein is not adversely affected by the herbicide.
c) Modification of the plant such that a novel protein/a novel RNA is introduced wherein the chemical structure of the protein or of the nucleic acid, such as of the RNA or the DNA, which structure is responsible for the herbicidal action of the low-molecular-weight substance, is modified so that, owing to the modified structure, a herbicidal action can no longer be developed, that is to say that the interaction of the herbicide with the target can no longer take place.
d) The function of the target is replaced by a novel gene introduced into the plant, creating what is known as an alternative pathway.
e) The function of the target is taken over by another gene which is present in the plant, or by its gene product.

The skilled worker is familiar with alternative methods for identifying the homologous nucleic acids, for example in other plants, with similar sequences, such as, for example, using transposons. The present invention therefore also relates to the use of alternative insertion mutagenesis methods for the insertion of foreign nucleic acids into the nucleic acid sequences according to the invention in other plants.

The transgenic plants are generated by customary transformation methods which have likewise been described above, using one of the above-described embodiments of the expression cassettes according to the invention.

The expression efficacy of the recombinantly expressed MSM variant can be determined for example in vitro by shoot meristem propagation or by a germination test.

Moreover, an expression of the MSM gene, which has been modified in terms of nature and level, and its effect on the resistance to MSM or VMSM inhibitors can be tested on test plants in greenhouse experiments.

The invention is illustrated in greater detail by the examples which follow, which are not to be construed as limiting.

### General DNA manipulation and cloning methods

Cloning methods such as, for example, restriction cleavages, agarose gel electrophoreses, purification of DNA fragments, transfer of nucleic acids to nitrocellulose and nylon membranes, linking DNA fragments, transformation of Escherichia coli cells, growing of bacteria and sequence analysis of recombinant DNA were carried out as described by Sambrook et al. (1989) (Cold Spring Harbor Laboratory Press: ISBN 0-87969-309-6) and Ausubel, F.M. et al., Current Protocols in Molecular Biology, Greene Publishing Assoc. and Wiley-Interscience (1994); ISBN 0-87969-309-6.

Molecular-biological standard methods for plants and plant transformation methods are described in Schultz et al., Plant Molecular Biology Manual, Kluwer Academic Publishers (1998), Reither et al., Methods in Arabidopsis Research, World scientific press (1992) and Arabidopsis: A Laboratory Manual (2001), ISBN 0-87969-573-0.

The bacterial strains used hereinbelow (E. coli DH5a, XL-1 blue, BL21 DE(3)) were obtained from Stratagene, BRL Gibco or Invitrogen, Carlsberg, CA. The vectors used for cloning were pCR T7CT TOPO, pCR T7/NT TOPO and pCR 2.1 TOPO from Invitrogen and pUC 19 from Amersham Pharmacia (Freiburg) and the vector pBinAR (Höfgen and Willmitzer, Plant Science 66, 1990, 221-230).

### Example 1: Generation of a cDNA library in the plant transformation vector

To generate a cDNA library (hereinbelow termed "binary cDNA library") in a vector which can be used directly for transforming plants, mRNA was isolated from a variety of plant tissues and transcribed into double-stranded cDNA using the TimeSaver cDNA Synthese Kit (Amersham Pharmacia Biotech, Freiburg). The cDNA first-strand synthesis was carried out using T₁₂₋₁₈ oligonucleotides following the manufacturer's instructions. After size fractionation and the ligation of EcoRI-Notl adapters following the manufacturer's instructions and filling up the overhangs with Pfu DNA polymerase (Stratagene), the cDNA population was normalized. The method of Kohci et al, 1995, Plant Journal 8, 771-776 was followed, the cDNA being amplified by PCR with the oligonucleotide N1 under the conditions given in Table 1.

**Table 1**

| Temperature [°C] | Time [sec] | Number of cycles |
|---|---|---|
| 94 | 300 | 1 |
| 94 | 8 | 10 |
| 52 | 60 | |
| 72 | 180 | |
| 94 | 8 | 10 |
| 50 | 60 | |
| 72 | 180 | |
| 94 | 8 | 10 |
| 48 | 60 | |
| 72 | 180 | |
| 72 | 420 | 1 |

The resulting PCR product was bound to the column matrix of the PCR purification kit (Qiagen, Hilden) and eluted with 300 mM NaP buffer, pH 7.0, 0.5 mM EDTA, 0.04% SDS. The DNA was denatured for 5 minutes in a boiling water bath and subsequently renatured for 24 hours at 60°C. 50 µl of the DNA were applied to a hydroxylapatite column and the column was washed 3 times with 1 ml of 10 mM NaP buffer, pH 6.8. The bound single-stranded DNA was eluted with 130 mM NaP buffer, pH 6.8, precipitated with ethanol and dissolved in 40 µl of water. 20 µl of growth were used for a further PCR amplification as described above. After further ssDNA concentration, a third PCR amplification was carried out as described above.

The plant transformation vector for taking up the cDNA population which had been generated as described above was generated via restriction enzyme cleavage of the vector pUC18 with SbfI and BamHI, purification of the vector fragment followed by filling up the overhangs with Pfu DNA polymerase and relegation with T4 DNA ligase (Stratagene). The resulting construct is hereinbelow termed pUC18Sbfl-.

The vector pBinAR was first cleaved with Notl, after the ends were filled up and the vector was relegated, cleaved with Sbfl, after the ends were filled up relegated and subsequently cleaved with EcoRI and HindIII. The resulting fragment was ligated into a derivative of the binary plant transformation vector pPZP (Hajdukiewicz,P, Svab, Z, Maliga, P., (1994) Plant Mol Biol 25:989-994) which makes possible the transformation of plants by means of agrobacterium and mediates kanamycin resistance in transgenic plants. The construct generated thus is hereinbelow termed pSun12/35S.

pUC18Sbfl- was used as template in a polymerase chain reaction (PCR) with the oligonucleotides V1 and V2 (see Table 2) and Pfu DNA polymerase. The resulting fragment was ligated into the Smal-cut pSun12/35S, giving rise to pSunblues2. Following cleavage with NotI, dephosphorylation with shrimp alkaline phosphatase (Roche Diagnostics, Mannheim) and purification of the vector fragment, pSunblues2 was ligated with the normalized, likewise Notl-cut cDNA population. Following transformation into E. coli XI-1 blue (Stratagene), the resulting clones were deposited into microtiter plates. The binary cDNA library comprises cDNAs in "sense" and in "antisense" orientation under the control of the cauliflower mosaic virus 35s promoter, and, after transformation into tobacco plants, these cDNAs can, accordingly, lead to "cosuppression" and "antisense" effects.

**Table 2: Oligonucleotides used**

| Oligonucleotide | Nucleic acid sequence |
|---|---|
| N1 | 5'-AGAATTCGCGGCCGCT-3' (SEQ ID NO:11) |
| V1 (PWL93not) | 5'-CTCATGCGGCCGCGCGCAACGCAATTAATGTG-3' (SEQ ID NO:12) |
| V2 (pWL92) | 5'-TCATGCGGCCGCGAGATCCAGTTCGATGTAAC-3' (SEQ ID NO:13) |
| G1 (35S) | 5'-GTGGATTGATGTGATATCTCC-3' (SEQ ID NO:14) |
| G2 (OCS) | 5'-GTAAGGATCTGAGCTACACAT-3' (SEQ ID NO:15) |

### Example 2: Transformation and analysis of tobacco plants

Selected clones of the binary cDNA library were transformed into Agrobacterium tumefaciens C58C1:pGV2260 (Deblaere et al., Nucl. Acids. Res. 13(1984), 4777-4788) and incubated with Streptomycin/Spectinomycin selection. The material used for the transformation of tobacco plants (Nicotiana tabacum cv. Samsun NN) with the binary clone NT006075002r was an overnight culture of a positively transformed agrobacterial colony diluted with YEB medium to OD600 = 0.8-1.6. Leaf discs of sterile plants (approx. 1 cm² each) were incubated for 5-10 minutes with the agrobacterial dilution in a Petri dish. This was followed by incubation in the dark for 2 days at 25°C on Murashige-Skoog medium (Physiol. Plant. 15(1962), 473) supplemented with 2% sucrose (2MS medium) and 0.8% Bacto agar. The cultivation was continued after 2 days at a 16-hour-light/8-hour-darkness photoperiod and continued in a weekly rhythm on MS medium supplemented with 500 mg/l Claforan (cefotaxime-sodium), 50 mg/l kanamycin, 1mg/l benzylaminopurin (BAP), 0.2 mg/l naphthylacetic acid and 1.6 g/l glucose. Regenerated shoots were transferred onto a 2MS medium supplemented with kanamycin and Claforan. Transgenic plants of line E_0000018240 were generated in this manner.

After the shoots had been transferred into soil, the plants were observed for 1-20 weeks in the greenhouse for the manifestation of phenotypes. It emerged that transgenic plants of line E_0000018240 were similar in phenotype. These plants showed pronounced growth retardation over comparative plants, pronounced foliar chloroses and necroses and some of them died within a few days.

The integration of the clone cDNA into the genome of the transgenic lines was detected via PCR with the oligonucleotides G1 and G2 (see Table 1 in Example 1) and genomic DNA prepared from the transgenic lines in question. To this end, TAKARA Taq DNA polymerase was preferably employed for this purpose, following the manufacturer's instructions (MoBiTec, Göttingen). The cDNA clone of the binary cDNA library, which clone had been used for the transformation, acted as template for a PCR reaction as the positive control. PCR products with an identical size or, if appropriate, identical cleavage patterns which were obtained after cleavage with a variety of restriction enzymes acted as proof that the corresponding cDNA had been integrated. In this manner, the insert of clone NT006075002r was detected in the transgenic plants with the abovementioned phenotypes.

### Example 3: Sequence analysis of the clone

The cDNA insert of clone NT006075002r, whose transformation into tobacco plants resulted in the abovementioned phenotypes, was sequenced.

The cDNA of NT006075002r (SEQ ID NO:9) has a length of 910 bp and comprises an open reading frame of 600 bp which encodes a polypeptide of 200 amino acids (SEQ ID NO:10). The corresponding full-length clone was identified by SEQ ID NO:9 (SEQ ID NO:1; Genbank Acc. No. X94968).

Moreover, SEQ ID NO:1 has significant identity with "apg1" from Arabidopsis thaliana (SEQ ID NO:3), so that it can be assumed that the encoded proteins have an identical function.

NT006075002r is thus a partial cDNA clone which encodes the C terminal portion of an enzyme from tobacco which is homologous to Arabidopsis thaliana 2-methyl-6-solanylbenzoquinone methyltransferase.

It was thus demonstrated for the first time and in a surprising manner that 2-methyl-6-solanylbenzoquinone methyltransferase is essential for plants and that even slightly reduced expression results in the abovementioned damage symptoms.

### Example 4: Expression in E. coli

To demonstrate the MSBQ-MT enzyme activity of the enzyme encoded by SEQ ID NO:1 and to generate sufficient 2-methyl-6-solanylbenzoquinone methyltransferase enzyme activity for HTS applications, fragments of the 2-methyl-6-solanylbenzoquinone methyltransferase cDNAs of SEQ ID NO:1 and SEQ ID NO:3 were amplified by polymerase chain reaction (PCR), and expression vectors were cloned. To this end, the cDNAs and cDNA libraries of Nicotiana tabacum and Arabidopsis thaliana were employed as templates for PCR with Pfu or Pfu-ultra polymerase (Stratagene, Heidelberg, Germany). Table 1 shows the oligonucleotides used and the fragments generated therewith. The resulting cDNA fragments were cloned into pCRT7/CT TOPO TA vectors (Invitrogen, Karlsruhe, Germany) and sequenced for the desired sequence identity for verification purposes. The resulting expression plasmids were transformed into E.coli BL-21 (DE3)RIL (Stratagene) strains. The transformed strains were grown overnight with shaking in liquid LB medium supplemented with 100 µg/ml ampicillin and 50 µg/ml chloramphenicol at 37°C. Large-volume day cultures were inoculated to OD600 = 0.1 and grown until an OD600 of 0.4-0.7 had been reached. At this point in time, the cultures were induced with 1 mM IPTG and grown for a further 2.5 hours. The analysis of cell extracts of the expression cultures by means of SDS polyacrylamide gel electrophoresis demonstrated that in particular the full-length 2-methyl-6-solanylbenzoquinone methyltransferase can only be expressed in a small amount, but that the N- and C-terminally truncated forms are expressed in markedly higher amounts. The elimination of the C-terminally predicted transmembrane domains is especially advantageous.

A purification is possible via the fused His tags using metal chelate matrices such as, for example, Hi-Trap columns (Pharmacia, Uppsala, Sweden).

| | Primer | | C Terminus | N Terminus*** |
|---|---|---|---|---|
| No. | 5' (N terminal) | 3' (C terminal) | | |
| K1* | | | +His tag | full length |
| K2* | | | His tag | full length |
| K3* | ATGAGCAGCAGCGTGTCG (SEQ ID NO:20) | | -31 AS +His tag | -51 AS |
| K4* | ATGTGCAGCAGCAGCAGC (SEQ ID NO:22) | | -31 AS +His tag | -49 AS |
| K5* | ATGTGCAGCAGCAGCAGC (SEQ ID NO:24) | | -31 AS | -49 AS |
| K6* | ATGAGCAGCAGCGTGTCG (SEQ ID NO:26) | | full length | (-51 AS) ab serine |
| K7* | ATGAGCAGCAGCGTGTCG (SEQ ID NO:28) | | full length +His tag | (-51 AS) ab serine |
| K8* | ATGTGCAGCAGCAGCAGC (SEQ ID NO:30) | | full length +His tag | -49 AS |
| K9* | ATGAGCAGCAGCGTGTCG (SEQ ID NO:32) | | -31 AS | (-51 AS) ab serine |
| K10** | ATGTGCAGCAGCAGCAGC (SEQ ID NO:34) | | full length | -49 AS |

| | | | | |
|---|---|---|---|---|
| *) Template: cDNA library from Arabidopsis **) Template cDNA library from Nicotiana tabacum ***) AS = amino acid | | | | |

### Example 5: In vitro assay systems

2-Methyl-6-solanylbenzoquinone methyltransferase activity of optionally purified 2-methyl-6-solanylbenzoquinone methyltransferase expressed in E. coli, of Example 4, can be measured as described using radiolabeled S-adenosylmethionine and detection via thin-layer chromatography (analogously to Peddibhotla et al. 2003 Tetrahedron Letters 44, pp. 237-239), it being possible to employ the following substrates: 2-methyl-6-solanylbenzoquinol, 2-methyl-6-phytylbenzoquinol, 2-methyl-6-geranylgeranylbenzoquinol and 2-methylbenzoquinol derivatives with truncated prenyl chain in the 6-position.

## Claims

1. The use of a truncated 2-methyl-6-solanylbenzoquinone methyltransferase in a method for identifying herbicides wherein the amino acid sequence of the truncated 2-methyl-6-solanylbenzoquinone methyltransferase is encoded by
i) a nucleic acid sequence with the nucleic acid sequence shown in SEQ ID NO:5 or in SEQ ID NO:7; or
ii) a nucleic acid sequence which, owing to the degeneracy of the genetic code, can be derived from the amino acid sequence shown in SEQ ID NO:6 or in SEQ ID NO:8 by back translation.

2. An expression cassette comprising
a) genetic control sequences inoperable linkage with the nucleic acid sequence shown in SEQ ID NO:5 or in SEQ ID NO:7; or a nucleic acid sequence which, owing to the degeneracy of the genetic code, can be derived from the amino acid sequence shown in SEQ ID NO:6 or in SEQ ID NO:8 by back translation; or
b) genetic control sequences in operable linkage with the nucleic acid sequence shown in SEQ ID NO:5 or in SEQ ID NO:7; or a nucleic acid sequence which, owing to the degeneracy of the genetic code, can be derived from the amino acid sequence shown in SEQ ID NO:6 or in SEQ ID NO:8 by back translation and additional functional elements.

3. A vector comprising an expression cassette according to claim 2.

4. A nonhuman transgenic organism comprising an expression cassette according to claim 2 or a vector according to claim 3, selected among bacteria, yeasts, fungi, animal or plant cells.

5. A method for identifying herbicidally active substances, comprising the following steps:
i. bringing a polypeptide with the activity of a 2-methyl-6-solanylbenzoquinone methyltransferase into contact with one or more test compounds under conditions which permit the binding of the test compound(s) to the polypeptide with the activity of a 2-methyl-6-solanylbenzoquinone methyltransferase wherein the polypeptide is encoded by a nucleic acid sequence with the nucleic acid sequence shown in SEQ ID NO:5 or in SEQ ID NO:7; or a nucleic acid sequence which, owing to the degeneracy of the genetic code, can be derived from the amino acid sequence shown in SEQ ID NO:6 or in SEQ ID NO:8 by back translation; and
ii. detecting whether the test compound reduces or blocks the activity of the polypeptide with the activity of a 2-methyl-6-solanylbenzoquinone methyltransferase of i).

6. A method according to claim 5, wherein a test compound which reduces or blocks the activity of 2-methyl-6-solanylbenzoquinone methyltransferase is selected.

7. The method according to claim 6, which comprises
i. either expressing, in a nonhuman transgenic organism, a truncated 2-methyl-6-solanylbenzoquinone methyltransferase which is encoded by
i) a nucleic acid sequence with the nucleic acid sequence shown in SEQ ID NO:5. or in SEQ ID NO:7; or
ii) a nucleic acid sequence which, owing to the degeneracy of the genetic code, can be derived from the amino acid sequence shown in SEQ ID NO:6 or in SEQ ID NO:8 by back translation;
ii. bringing the 2-methyl-6-solanylbenzoquinone methyltransferase of step i) in the cell digest of the nonhuman transgenic organism, in partially or homogeneously purified form, into contact with a test compound; and
iii. selecting a test compound which reduces or blocks the activity of the 2-methyl-6-solanylbenzoquinone methyltransferase of step ii).

8. The method according to claim 6, which comprises the following steps:
i. generating a nonhuman transgenic organism comprising at least one nucleic acid sequence encoding a truncated 2-methyl-6-solanylbenzoquinone methyltransferase which is encoded by
i) a nucleic acid sequence with the nucleic acid sequence shown in SEQ ID NO:5. or in SEQ ID NO:7; or
ii) a nucleic acid sequence which, owing to the degeneracy of the genetic code, can be derived from the amino acid sequence shown in SEQ ID NO:6 or in SEQ ID NO:8 by back translation,
in which organism 2-methyl-6-solanylbenzoquinone- methyltransferase is overexpressed;
ii. applying a test substance to the nonhuman transgenic organism of i) and to a nontransgenic organism of the same type;
iii. determining the growth or the viability of the transgenic and the nontransgenic organisms after application of the test substance; and
iv. selecting test substances which bring about a reduced growth or a reduced viability of the nontransgenic organism in comparison with the growth of the nonhuman transgenic organism.

9. A method for identifying growth-regulatory substances, which comprises the following steps;
i. generation of a transgenic plant comprising a nucleic acid sequence encoding a truncated 2-methyl-6-solanylbenzoquinone methyltransferase which is encoded by
i) a nucleic acid sequence with the nucleic acid sequence shown in SEQ ID NO:5. or in SEQ ID NO:7; or
ii) a nucleic acid sequence which, owing to the degeneracy of the genetic code, can be derived from the amino acid sequence shown in SEQ ID NO:6 or in SEQ ID NO:8 by back translation,
in which the truncated plant 2-methyl-6-solanylbenzoquinone methyltransferase is overexpressed;
ii. applying a test substance to the transgenic plant of i) and to a nontransgenic plant of the same type;
iii. determining the growth of the viability of the transgenic and of the nontransgenic plants after application of the test substance; and
iv. selecting test substances which bring about a modified growth of the nontransgenic plant in comparison with the growth of the transgenic plant.

10. The method according to any of claims 5 to 9, wherein the substances are identified in a high-throughput screening.

11. A support having one or more of the expression cassettes according to claim 2 or one or more vectors according to claim 3

12. The method according to any of claims 5 to 10, wherein the substances are identified in a high-throughput screening using a support according to claim 11.

13. A method for generating nucleic acid sequences which encode a truncated 2-methyl-6-solanylbenzoquinone methyltransferase which are not inhibited by a herbicidally active compound identified by one of the methods according to any of claims 5 to 9 and 11 or a compound with growth-regulatory activity identified via the method according to any of claims 10 and 11, where the nucleic acid sequence comprises
i) a functional equivalent of the nucleic acid sequence SEQ ID NO:5 or SEQ ID NO:7 which can be derived by back translating an amino acid sequence with SEQ ID NO:6 or SEQ ID NO:8 ; which comprises the following process steps: a) expression, in a heterologous system or in a cell-free system, of the proteins encoded by the nucleic acids of i) above; b) randomized or site-directed mutagenesis of the protein by modification of the nucleic acid ; c) measuring the interaction of the modified gene product with the herbicide; d) identification of derivatives of the protein which exhibit a lesser degree of interaction; e) testing the biological activity of the protein after application of the herbicide; and f) selection of the nucleic acid sequences which exhibit a modified biological activity toward the herbicide.

14. The method according to claim 13, wherein the sequences selected according to claim 13 f) are introduced into a plant.

15. The method for generating transgenic plants which are resistant to substances according to claim 12, which comprises overexpressing, in these plants, at least one nucleic acid sequence which encodes a truncated 2-methyl-6-solanylbenzoquinone methyltransferase, wherein the nucleic acid sequence comprises a nucleic acid with the nucleic acid sequence shown in SEQ ID NO:5 or in SEQ ID NO:7; or a nucleic acid sequence which, owing to the degeneracy of the genetic code, can be derived from the amino acid sequence shown in SEQ ID NO:6 or in SEQ ID NO:8 by back translation.

16. A transgenic plant which comprises at least one nucleic acid sequence which encodes a truncated 2-methyl-6-solanylbenzoquinone methyltransferase according to claim 1 which is overexpressed, wherein the nucleic acid sequence comprises
i. a nucleic acid with the nucleic acid sequence shown in SEQ ID NO:5 or in SEQ ID NO:7; or
ii. a nucleic acid sequence which, owing to the degeneracy of the genetic code, can be derived from the amino acid sequence shown in SEQ ID NO:6 or in SEQ ID NO:8 by back translation .

## Patentansprüche

1. Verwendung einer verkürzten 2-Methyl-6-Solanylbenzochinon-Methyltransferase in einem Verfahren zur Identifizierung von Herbiziden, bei der die Aminosäuresequenz der verkürzten 2-Methyl-6-Solanylbenzochinon-Methyltransferase kodiert wird von
i) einer Nukleinsäuresequenz mit der in SEQ ID NO:5 oder in SEQ ID NO:7 dargestellten Nukleinsäuresequenz; oder
ii) einer Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt.

2. Expressionskassette, umfassend
a) genetische Kontrollsequenzen in funktioneller Verknüpfung mit der in SEQ ID NO:5 oder in SEQ ID NO:7 dargestellten Nukleinsäuresequenz oder mit einer Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt; oder
b) genetische Kontrollsequenzen in funktioneller Verknüpfung mit der in SEQ ID NO:5 oder in SEQ ID NO:7 dargestellten Nukleinsäuresequenz oder mit einer Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt und zusätzliche Funktionselemente.

3. Vektor, umfassend eine Expressionskassette gemäß Anspruch 2.

4. Nicht humaner, transgener Organismus, umfassend eine Expressionskassette gemäß Anspruch 2 oder einen Vektor gemäß Anspruch 3, ausgewählt aus Bakterien, Hefen, Pilzen, tierischen oder pflanzlichen Zellen.

5. Verfahren zur Identifizierung von Substanzen mit herbizider Wirkung, umfassend die folgenden Schritte:
i. Inkontaktbringen eines Polypeptids mit der Aktivität einer 2-Methyl-6-Solanylbenzochinon-Methyltransferase mit einer oder mehreren Testverbindungen unter Bedingungen, welche die Bindung der Testverbindung(en) an das Polypeptid mit der Aktivität einer 2-Methyl-6-Solanylbenzochinon-Methyltransferase erlauben, wobei das Polypeptid kodiert wird von einer Nukleinsäuresequenz mit der in SEQ ID NO:5 oder in SEQ ID NO:7 dargestellten Nukleinsäuresequenz; oder einer Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt; und
ii. Nachweis, ob die Testverbindung die Aktivität des Polypeptids mit der Aktivität einer 2-Methyl-6-Solanylbenzochinon-Methyltransferase aus i) reduziert oder blockiert.

6. Verfahren nach Anspruch 5, bei dem eine Testverbindung selektiert wird, welche die Aktivität der 2-Methyl-6-Solanylbenzochinon-Methyltransferase reduziert oder blockiert.

7. Verfahren nach Anspruch 6, bei dem
i. eine verkürzte 2-Methyl-6-Solanylbenzochinon-Methyltransferase, welche kodiert wird von
i) einer Nukleinsäuresequenz mit der in SEQ ID NO:5 oder in SEQ ID NO:7 dargestellten Nukleinsäuresequenz; oder
ii) einer Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt, entweder in einem nicht humanen transgenen Organismus exprimiert wird;
ii. die 2-Methyl-6-Solanylbenzochinon-Methyltransferase aus Schritt i) im Zellaufschluß des nicht humanen transgenen Organismus, in partieller gereinigter Form oder in zur Homogenität gereinigten Form, mit einer Testverbindung in Kontakt gebracht wird; und
iii. eine Testverbindung selektiert wird, welche die Aktivität der 2-Methyl-6-Solanylbenzochinon-Methyltransferase aus Schritt ii) reduziert oder blockiert.

8. Verfahren nach Anspruch 6, welches folgende Schritte umfaßt:
i. Herstellung eines nicht humanen transgenen Organismus, enthaltend mindestens eine Nukleinsäuresequenz, kodierend für eine verkürzte 2-Methyl-6-Solanylbenzochinon-Methyltransferase, welche kodiert wird von
i) einer Nukleinsäuresequenz mit der in SEQ ID NO:5 oder in SEQ ID NO:7 dargestellten Nukleinsäuresequenz; oder
ii) einer Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt, in welchem 2-Methyl-6-Solanylbenzochinon-Methyltransferase überexprimiert wird;
ii. Aufbringen einer Testsubstanz auf den nicht humanen transgenen Organismus nach i) und auf einen nicht-transgenen Organismus des gleichen Genotyps; und
iii. Bestimmen des Wachstums oder der Überlebensfähigkeit des transgenen und des nicht-transgenen Organismus nach der Aufbringung der Testsubstanz; und
iv. Selektion von Testsubstanzen, die ein vermindertes Wachstum oder eine eingeschränkte Überlebensfähigkeit des nicht-transgenen Organismus bewirken verglichen mit dem Wachstum des nicht humanen transgenen Organismus.

9. Verfahren zur Identifizierung von Substanzen mit wachstumsregulatorischer Wirkung, welches die folgenden Schritte umfaßt:
i. Herstellung einer transgenen Pflanze, enthaltend eine Nukleinsäuresequenz, kodierend für eine verkürzte 2-Methyl-6-Solanylbenzochinon-Methyltransferase, welche codiert wird von
i) einer Nukleinsäuresequenz mit der in SEQ ID NO:5 oder in SEQ ID NO:7 dargestellten Nukleinsäuresequenz; oder
ii) einer Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt, in welcher die verkürzte 2-Methyl-6-Solanylbenzochinon-Methyltransferase überexprimiert wird;
ii. Aufbringen einer Testsubstanz auf die transgene Pflanze nach i) und auf eine nichttransgene Pflanze der gleichen Sorte;
iii. Bestimmen des Wachstums oder der Überlebensfähigkeit der transgenen und der nicht-transgenen Pflanze nach dem Aufbringen der Testsubstanz; und
iv. Selektion von Testsubstanzen, die ein verändertes Wachstum der nicht-transgenen Pflanze bewirken verglichen mit dem Wachstum der transgenen Pflanze.

10. Verfahren nach einem der Ansprüche 5 bis 9, bei dem die Identifizierung der Substanzen wie in einem High-Throughput-Screening durchgeführt wird.

11. Träger mit einer oder mehreren Expressionskassetten gemäß Anspruch 2 oder einem oder mehreren Vektoren gemäß Anspruch 3.

12. Verfahren nach einem der Ansprüche 5 bis 10, bei dem die Identifizierung der Substanzen in einem High-Throughput-Screening durchgeführt wird unter Verwendung eines Trägers gemäß Anspruch 11.

13. Verfahren zur Erzeugung von Nukleinsäuresequenzen, welche für eine verkürzte 2-Methyl-6-Solanylbenzochinon-Methyltransferase kodieren, die durch eine Verbindung mit herbizider Wirkung, identifiziert über eines der Verfahren gemäß einem der Ansprüche 5 bis 9 und 11 oder durch eine Verbindung mit wachstumsregulatorischer Wirkung, identifiziert über das Verfahren gemäß einem der Ansprüche 10 und 11 nicht inhibitiert werden, wobei die Nukleinsäuresequenz
i) ein funktionelles Äquivalent der Nukleinsäuresequenz SEQ ID NO:5 oder SEQ ID NO:7, das sich durch Rückübersetzung einer Aminosäuresequenz mit SEQ ID NO:6 oder SEQ ID NO:8 ableiten läßt, umfaßt, umfassend folgende Prozeßschritte: a) Expression der von den Nukleinsäuren gemäß i) oben kodierten Proteine in einem heterologen System oder in einem zellfreien System; b) randomisierte oder gerichtete Mutagenese des Proteins durch Modifikation der Nukleinsäure; c) Messung der Interaktion des veränderten Genprodukts mit dem Herbizid; d) Identifizierung von Derivaten des Proteins, die eine geringere Interaktion aufweisen; e) Testung der biologischen Aktivität des Proteins nach Applikation des Herbizides; und f) Auswahl der Nukleinsäuresequenzen, die eine veränderte biologische Aktivität gegenüber dem Herbizid aufweisen.

14. Verfahren nach Anspruch 13, bei dem die gemäß Anspruch 13 f) ausgewählten Sequenzen in eine Pflanze eingebracht werden.

15. Verfahren zur Erzeugung transgener Pflanzen, die gegen Substanzen gemäß Anspruch 12 resistent sind, bei dem in diesem Pflanzen mindestens eine Nukleinsäuresequenz, welche für eine verkürzte 2-Methyl-6-Solanylbenzochinon-Methyltransferase kodiert, überexprimiert wird, wobei die Nukleinsäuresequenz eine Nukleinsäure mit der in SEQ ID NO:5 oder SEQ ID NO:7 dargestellten Nukleinsäuresequenz oder eine Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt, umfaßt.

16. Transgene Pflanze, umfassend mindestens eine für eine verkürzte 2-Methyl-6-Solanylbenzochinon-Methyltransferase gemäß Anspruch 1 kodierende Nukleinsäuresequenz, die überexprimiert wird, wobei die Nukleinsäuresequenz
i. eine Nukleinsäure mit der in SEQ ID NO:5 oder in SEQ ID NO:7 dargestellten Nukleinsäuresequenz; oder
ii. eine Nukleinsäuresequenz, die sich aufgrund des degenerierten genetischen Codes durch Rückübersetzung aus der in SEQ ID NO:6 oder in SEQ ID NO:8 dargestellten Aminosäuresequenz ableiten läßt, umfaßt.

## Revendications

1. Utilisation d'une 2-méthyl-6-solanylbenzoquinone méthyltransférase tronquée dans un procédé pour identifier des herbicides dans laquelle la séquence d'acides aminés de la 2-méthyl-6-solanylbenzoquinone méthyltransférase tronquée est codée par
i) une séquence d'acide nucléique avec la séquence d'acide nucléique représentée dans SEQ ID NO : 5 ou dans SEQ ID NO : 7 ; ou
ii) une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse.

2. Cassette d'expression comprenant
a) des séquences de contrôle génétique en liaison fonctionnelle avec la séquence d'acide nucléique représentée dans SEQ ID NO : 5 ou dans SEQ ID NO : 7 ; ou une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse ; ou
b) des séquences de contrôle génétique en liaison fonctionnelle avec la séquence d'acide nucléique représentée dans SEQ ID NO : 5 ou dans SEQ ID NO : 7 ; ou une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse et des éléments fonctionnels supplémentaires.

3. Vecteur comprenant une cassette d'expression selon la revendication 2.

4. Organisme non humain transgénique comprenant une cassette d'expression selon la revendication 2 ou un vecteur selon la revendication 3, choisi parmi des bactéries, des levures, des champignons, des cellules animales ou végétales.

5. Procédé pour identifier des substances actives du point de vue herbicide, comprenant les étapes suivantes :
i. la mise en contact d'un polypeptide ayant l'activité d'une 2-méthyl-6-solanylbenzoquinone méthyltransférase avec un ou plusieurs composés de test dans des conditions qui permettent la liaison du ou des composés de test au polypeptide ayant l'activité d'une 2-méthyl-6-solanylbenzoquinone méthyltransférase, où le polypeptide est codé par une séquence d'acide nucléique avec la séquence d'acide nucléique représentée dans SEQ ID NO : 5 ou dans SEQ ID NO : 7 ; ou une séquence d'acide nucléique qui, en raison de la dégénérescence du code génétique, peut être dérivée de la séquence diacides aminés représentée dans SEQ ID NO : 6 ou dans SEQ ID NO : 8 par traduction inverse ; et
ii. l'opération consistant à détecter si le composé de test réduit ou bloque l'activité du polypeptide ayant l'activité d'une 2-méthyl-6-solanylbenzoquinone méthyltransférase de i).

6. Procédé selon la revendication 5, dans lequel un composé de test qui réduit ou bloque l'activité de 2-méthyl-6-solanylbenzoquinone méthyltransférase est sélectionné.

7. Procédé selon la revendication 6, lequel comprend
i. soit l'expression, dans un organisme non humain transgénique, d'une 2-méthyl-6-solanylbenzoquinone méthyltransférase tronquée qui est codée par
i) une séquence d'acide nucléique avec la séquence d'acide nucléique représentée dans SEQ ID NO : 5 ou dans SEQ ID NO : 7 ; ou
ii) une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse ;
ii. la mise en contact de la 2-méthyl-6-solanylbenzoquinone méthyltransférase de l'étape i) dans le produit de digestion de cellules de l'organisme non humain transgénique, sous forme partiellement purifiée ou purifiée de façon homogène, avec un composé de test ; et
iii. la sélection d'un composé de test qui réduit ou bloque l'activité de la 2-méthyl-6-solanylbenzoquinone méthyltransférase de l'étape ii).

8. Procédé selon la revendication 6, lequel comprend les étapes suivantes :
i. la création d'un organisme non humain transgénique comprenant au moins une séquence d'acide nucléique codant pour une 2-méthyl-6-solanylbenzoquinone méthyltransférase tronquée qui est codée par
i) une séquence d'acide nucléique avec la séquence d'acide nucléique représentée dans SEQ ID NO : 5 ou dans SEQ ID NO : 7 ; ou
ii) une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse, dans lequel organisme la 2-méthyl-6-solanylbenzoquinone méthyltransférase est surexprimée ;
ii. l'application d'une substance de test à l'organisme non humain transgénique de i) et à un organisme non transgénique du même type ;
iii. la détermination de la croissance ou de la viabilité des organismes transgénique et non transgénique après l'application de la substance de test ; et
iv. la sélection des substances de test qui conduisent à une croissance réduite ou une viabilité réduite de l'organisme non transgénique par rapport à la croissance de l'organisme non humain transgénique.

9. Procédé pour identifier des substances régulatrices de croissance, qui comprend les étapes suivantes :
i. la création d'une plante transgénique comprenant une séquence d'acide nucléique codant pour une 2-méthyl-6-solanylbenzoquinone méthyltransférase tronquée qui est codée par
i) une séquence d'acide nucléique avec la séquence d'acide nucléique représentée dans SEQ ID NO : 5 ou dans SEQ ID NO : 7 ; ou
ii) une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse, dans laquelle la 2-méthyl-6-solanylbenzoquinone méthyltransférase végétale tronquée est surexprimée ;
ii. l'application d'une substance de test à la plante transgénique de i) et à une plante non transgénique du même type ;
iii. la détermination de la croissance ou de la viabilité des plantes transgénique et non transgénique après l'application de la substance de test ; et
iv. la sélection des substances de test qui conduisent à une croissance modifiée de la plante non transgénique par rapport à la croissance de la plante transgénique.

10. Procédé selon l'une quelconque des revendications 5 à 9, dans lequel les substances sont identifiées dans un criblage à haut débit.

11. Support ayant une ou plusieurs des cassettes d'expression selon la revendication 2 ou un ou plusieurs vecteurs selon la revendication 3.

12. Procédé selon l'une quelconque des revendications 5 à 10, dans lequel les substances sont identifiées dans un criblage à haut débit à l'aide d'un support selon la revendication 11.

13. Procédé pour générer des séquences d'acide nucléique qui codent pour une 2-méthyl-6-solanylbenzoquinone méthyltransférase tronquée qui ne sont pas inhibées par un composé actif du point de vue herbicide identifié par l'un des procédés selon l'une quelconque des revendications 5 à 9 et 11 ou un composé ayant une activité régulatrice de croissance identifiée par le procédé selon l'une quelconque des revendications 10 et 11, où la séquence d'acide nucléique comprend
i) un équivalent fonctionnel de la séquence d'acide nucléique SEQ ID NO : 5 ou SEQ ID NO : 7 qui peut être dérivé par traduction inverse d'une séquence d'acides aminés avec SEQ ID NO : 6 ou SEQ ID NO : 8 ; qui comprend les étapes de procédé suivantes : a) l'expression, dans un système hétérologue ou un système sans cellules, des protéines codées par les acides nucléiques de i) ci-dessus ; b) la mutagenèse aléatoire ou dirigée de la protéine par modification de l'acide nucléique ; c) la mesure de l'interaction du produit génétique modifié avec l'herbicide ; d) l'identification de dérivés de la protéine qui présentent un degré moindre d'interaction ; e) le test de l'activité biologique de la protéine après application de l'herbicide ; et f) la sélection des séquences d'acide nucléique qui présentent une activité biologique modifiée vis-à-vis de l'herbicide.

14. Procédé selon la revendication 13, dans lequel les séquences sélectionnées selon la revendication 13 f) sont introduites dans une plante.

15. Procédé pour générer des plantes transgéniques qui sont résistantes à des substances selon la revendication 12, qui comprend la surexpression, dans ces plantes, d'au moins une séquence d'acide nucléique qui code pour une 2-méthyl-6-solanylbenzoquinone méthyltransférase tronquée, dans lequel la séquence d'acide nucléique comprend un acide nucléique avec la séquence d'acide nucléique représentée dans SEQ ID NO : 5 ou dans SEQ ID NO : 7 ; ou une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse.

16. Plante transgénique qui comprend au moins une séquence d'acide nucléique qui code pour une 2-méthyl-6-solanylbenzoquinone méthyltransférase tronquée selon la revendication 1 qui est surexprimée, dans laquelle la séquence d'acide nucléique comprend
i. un acide nucléique avec la séquence d'acide nucléique représentée dans SEQ ID NO : 5 ou dans SEQ ID NO : 7 ; ou
ii. une séquence d'acide nucléique qui, en raison de la dégénérescence du code génétique, peut être dérivée de la séquence d'acides aminés représentée dans SEQ ID NO : 6 ou dans SEQ ID NO : 8 par traduction inverse.


### SEQUENCE LISTING

<110> BASF Aktiengesellschaft
<120> 2-Methyl-6-solanylbenzoquinone methyltransferase as target for herbicides
<130> 20030911
<160> 35
<170> PatentIn version 3.1
<210> 1
   <211> 1355
   <212> DNA
   <213> Nicotiana tabacum
<220>
   <221> CDS
   <222> (110)..(1117)
   <223>
<400> 1
   cttttttttc cgcttctcct ccaaaatccc atcaaaattg ataagcttct cttctgaagc      60
   ttatcaaaac tatatgcagt aaaaaaaata acatcaaaaa tacatatcc atg gct tct     118
                                                          Met Ala Ser
                                                          1
   tca ata cta agt gga gct gaa aat ttc aag att ctt agt ggt att tct       166
   Ser Ile Leu Ser Gly Ala Glu Asn Phe Lys Ile Leu Ser Gly Ile Ser
       5                   10                  15
   cca tca gaa tta cac att aag tgt ttt cct caa aag ggt ctt gta aat       214
   Pro Ser Glu Leu His Ile Lys Cys Phe Pro Gln Lys Gly Leu Val Asn
   20                  25                  30                  35
   tac tca aga att cca aat acc aaa tca aga act cta aga aca aaa tgc       262
   Tyr Ser Arg Ile Pro Asn Thr Lys Ser Arg Thr Leu Arg Thr Lys Cys
                   40                  45                  50
   agt gta tca tct tca aga cca gct tca caa cca aga ttt ata caa cac       310
   Ser Val Ser Ser Ser Arg Pro Ala Ser Gln Pro Arg Phe Ile Gln His
   55 60 65
   aaa aaa gaa gca ttt tgg ttt tac aga ttc tta tct ata gta tat gac 358
   Lys Lys Glu Ala Phe Trp Phe Tyr Arg Phe Leu Ser Ile Val Tyr Asp
   70 75 80
   cat gtt ata aat cca ggt cat tgg act gaa gat atg aga gat gaa gca 406
   His Val Ile Asn Pro Gly His Trp Thr Glu Asp Met Arg Asp Glu Ala
   85 90 95
   ctt gaa cca gct gaa tta aac agt aga caa ttg caa gtt gtg gat gtt 454
   Leu Glu Pro Ala Glu Leu Asn Ser Arg Gln Leu Gln Val Val Asp Val
   100 105 110 115
   ggt ggt ggg act gga ttt act act ctt ggc att gtg aaa cat gtg gat 502
   Gly Gly Gly Thr Gly Phe Thr Thr Leu Gly Ile Val Lys His Val Asp
   120 125 130
   gct aag aat gtt aca att att gat caa tca cct cat caa ctt gcc aag 550
   Ala Lys Asn Val Thr Ile Ile Asp Gln Ser Pro His Gln Leu Ala Lys
   135 140 145
   gct aga gaa aag gaa cct ttg aaa gaa tgt aag ata ttg gaa gga gat 598
   Ala Arg Glu Lys Glu Pro Leu Lys Glu Cys Lys Ile Leu Glu Gly Asp
   150 155 160
   gct gag gat ttg cct ttt cct act gat act ttt gat aga tat gtt tct 646
   Ala Glu Asp Leu Pro Phe Pro Thr Asp Thr Phe Asp Arg Tyr Val Ser
   165 170 175
   gct gga agc att gag tat tgg ccc gat cca cag cgc ggt atc aag gaa 694
   Ala Gly Ser Ile Glu Tyr Trp Pro Asp Pro Gln Arg Gly Ile Lys Glu
   180 185 190 195
   gca tac cga gta ctg acc ata ggt ggt gtt gcc tgc tta ata ggt cct 742
   Ala Tyr Arg Val Leu Thr Ile Gly Gly Val Ala Cys Leu Ile Gly Pro
   200 205 210
   gtg tac ccg acg ttt tgg cta tct cgt ttc ttt gca gat atg tgg atg 790
   Val Tyr Pro Thr Phe Trp Leu Ser Arg Phe Phe Ala Asp Met Trp Met
   215 220 225
   ctc ttt cca aaa gaa gaa gaa tat ata gaa tgg ttc aaa aaa gct ggt 838
   Leu Phe Pro Lys Glu Glu Glu Tyr Ile Glu Trp Phe Lys Lys Ala Gly
   230 235 240
   ttc gct caa gtt aaa ctc aag agg att ggc cca aaa tgg tat cgt ggt 886
   Phe Ala Gln Val Lys Leu Lys Arg Ile Gly Pro Lys Trp Tyr Arg Gly
   245 250 255
   gtc cgt cgc cat ggc ttg atc atg ggt tgt tct gtg act ggt gtc aag 934
   Val Arg Arg His Gly Leu Ile Met Gly Cys Ser Val Thr Gly Val Lys
   260 265 270 275
   cca tat ttt ggg gaa tct ccg ttg cag ctc ggc ccg aag gtt gag gat 982
   Pro Tyr Phe Gly Glu Ser Pro Leu Gln Leu Gly Pro Lys Val Glu Asp
   280 285 290
   gtg agc aag cct gta aac cca ttc gca ttt ctc gtg cga ttc ctc ctc 1030
   Val Ser Lys Pro Val Asn Pro Phe Ala Phe Leu Val Arg Phe Leu Leu
   295 300 305
   ggc ata act gct gca act tat tac gtg ctc gtt cca ata tac atg tgg 1078
   Gly Ile Thr Ala Ala Thr Tyr Tyr Val Leu Val Pro Ile Tyr Met Trp
       310                 315                 320
   ctc aag gat caa atc acc ccg aaa ggt cag cca atc tga acaataagaa      1127
   Leu Lys Asp Gln Ile Thr Pro Lys Gly Gln Pro Ile
       325                 330                 335
   gaacgtcaat ccaaagagaa gctctccaag cattctgttt gagagtacac cagtgaccac   1187
   aaatctatca cggaacaaga aagtttttgg cgtcgttgca agggtgaatt tgttgcttta   1247
   gtttgttagt tttgcagcct tagaaagggc cttttgtaaa gtttaatttc atggtaaaac   1307
   ctagaaatca ttgtgactat tttctagttg tataatctat cagtcatg                1355
<210> 2
   <211> 335
   <212> PRT
   <213> Nicotiana tabacum
<400> 2
   Met Ala Ser Ser Ile Leu Ser Gly Ala Glu Asn Phe Lys Ile Leu Ser
   1               5                   10                  15
   Gly Ile Ser Pro Ser Glu Leu His Ile Lys Cys Phe Pro Gln Lys Gly
               20                  25                  30
   Leu Val Asn Tyr Ser Arg Ile Pro Asn Thr Lys Ser Arg Thr Leu Arg
           35                  40                  45
   Thr Lys Cys Ser Val Ser Ser Ser Arg Pro Ala Ser Gln Pro Arg Phe
       50                  55                  60
   Ile Gln His Lys Lys Glu Ala Phe Trp Phe Tyr Arg Phe Leu Ser Ile
   65                  70                  75                  80
   Val Tyr Asp His Val Ile Asn Pro Gly His Trp Thr Glu Asp Met Arg
                   85                  90                  95
   Asp Glu Ala Leu Glu Pro Ala Glu Leu Asn Ser Arg Gln Leu Gln Val
               100                 105                 110
   Val Asp Val Gly Gly Gly Thr Gly Phe Thr Thr Leu Gly Ile Val Lys
           115                 120                 125
   His Val Asp Ala Lys Asn Val Thr Ile Ile Asp Gln Ser Pro His Gln
       130                 135                 140
   Leu Ala Lys Ala Arg Glu Lys Glu Pro Leu Lys Glu Cys Lys Ile Leu
   145 150 155 160
   Glu Gly Asp Ala Glu Asp Leu Pro Phe Pro Thr Asp Thr Phe Asp Arg
   165 170 175
   Tyr Val Ser Ala Gly Ser Ile Glu Tyr Trp Pro Asp Pro Gln Arg Gly
   180 185 190
   Ile Lys Glu Ala Tyr Arg Val Leu Thr Ile Gly Gly Val Ala Cys Leu
   195 200 205
   Ile Gly Pro Val Tyr Pro Thr Phe Trp Leu Ser Arg Phe Phe Ala Asp
   210 215 220
   Met Trp Met Leu Phe Pro Lys Glu Glu Glu Tyr Ile Glu Trp Phe Lys
   225 230 235 240
   Lys Ala Gly Phe Ala Gln Val Lys Leu Lys Arg Ile Gly Pro Lys Trp
   245 250 255
   Tyr Arg Gly Val Arg Arg His Gly Leu Ile Met Gly Cys Ser Val Thr
   260 265 270
   Gly Val Lys Pro Tyr Phe Gly Glu Ser Pro Leu Gln Leu Gly Pro Lys
   275 280 285
   Val Glu Asp Val Ser Lys Pro Val Asn Pro Phe Ala Phe Leu Val Arg
   290 295 300
   Phe Leu Leu Gly Ile Thr Ala Ala Thr Tyr Tyr Val Leu Val Pro Ile
   305 310 315 320
   Tyr Met Trp Leu Lys Asp Gln Ile Thr Pro Lys Gly Gln Pro Ile
   325 330 335
<210> 3
   <211> 1017
   <212> DNA
   <213> Arabidopsis thaliana
<220>
   <221> CDS
   <222> (1)..(1017)
   <223>
<400> 3
   atg gcc tct ttg atg ctc aac ggg gcc att acc ttc ccc aaa ggt tta       48
   Met Ala Ser Leu Met Leu Asn Gly Ala Ile Thr Phe Pro Lys Gly Leu
   1               5                   10                  15
   ggt tcc cct ggt tcc aat ttg cat gcc aaa tcg att cct cgg ccg acc       96
   Gly Ser Pro Gly Ser Asn Leu His Ala Lys Ser Ile Pro Arg Pro Thr
               20                  25                  30
   tta ctc tca gtt acc cga acc tcc aca cct aga ctc tcg gtg gct act      144
   Leu Leu Ser Val Thr Arg Thr Ser Thr Pro Arg Leu Ser Val Ala Thr
           35                  40                  45
   aaa tgc agc agc agc agc gtg tcg tct tcc cgg cca tcg gcg caa cct      192
   Lys Cys Ser Ser Ser Ser Val Ser Ser Ser Arg Pro Ser Ala Gln Pro
       50                  55                  60
   agg ttc att cag cac aag aag gag gct tac tgg ttc tac agg ttc tta      240
   Arg Phe Ile Gln His Lys Lys Glu Ala Tyr Trp Phe Tyr Arg Phe Leu
   65                  70                  75                  80
   tcc atc gta tac gac cat gtc atc aat cct ggg cat tgg acc gag gat      288
   Ser Ile Val Tyr Asp His Val Ile Asn Pro Gly His Trp Thr Glu Asp
                   85                  90                  95
   atg aga gac gac gct ctt gag cca gcg gat ctc agc cat ccg gac atg      336
   Met Arg Asp Asp Ala Leu Glu Pro Ala Asp Leu Ser His Pro Asp Met
               100                 105                 110
   cga gtg gtc gat gtc ggc ggc gga act ggt ttc act act ctg ggc ata      384
   Arg Val Val Asp Val Gly Gly Gly Thr Gly Phe Thr Thr Leu Gly Ile
           115                 120                 125
   gtc aag aca gtg aag gcc aag aat gtg acc att ctg gac cag tcg cca      432
   Val Lys Thr Val Lys Ala Lys Asn Val Thr Ile Leu Asp Gln Ser Pro
       130                 135                 140
   cat cag ctg gcc aaa gca aag caa aag gag ccg ttg aaa gaa tgc aag      480
   His Gln Leu Ala Lys Ala Lys Gln Lys Glu Pro Leu Lys Glu Cys Lys
   145                 150                 155                 160
   atc gtc gag gga gat gct gag gat ctt cct ttt cca acc gat tat gct      528
   Ile Val Glu Gly Asp Ala Glu Asp Leu Pro Phe Pro Thr Asp Tyr Ala
                   165                 170                 175
   gac aga tac gtt tct gct gga agc att gag tac tgg ccg gac ccg cag      576
   Asp Arg Tyr Val Ser Ala Gly Ser Ile Glu Tyr Trp Pro Asp Pro Gln
               180                 185                 190
   agg gga ata agg gaa gcg tac agg gtt ctc aag atc ggt ggc aaa gcg      624
   Arg Gly Ile Arg Glu Ala Tyr Arg Val Leu Lys Ile Gly Gly Lys Ala
           195                 200                 205
   tgt ctc atc ggc cct gtc tac cca acc ttc tgg ctc tct cgc ttc ttt      672
   Cys Leu Ile Gly Pro Val Tyr Pro Thr Phe Trp Leu Ser Arg Phe Phe
       210                 215                 220
   tct gat gtc tgg atg ctc ttc ccc aag gag gaa gag tac att gag tgg      720
   Ser Asp Val Trp Met Leu Phe Pro Lys Glu Glu Glu Tyr Ile Glu Trp
   225                 230                 235                 240
   ttc aag aat gcc ggt ttc aag gac gtt cag ctc aag agg att ggc ccc      768
   Phe Lys Asn Ala Gly Phe Lys Asp Val Gln Leu Lys Arg Ile Gly Pro
                   245                 250                 255
   aag tgg tac cgt ggt gtt cgc agg cac ggc ctt atc atg gga tgt tct      816
   Lys Trp Tyr Arg Gly Val Arg Arg His Gly Leu Ile Met Gly Cys Ser
               260                 265                 270
   gtc act ggt gtt aaa cct gcc tcc ggt gat tct cct ctc cag ctt ggt      864
   Val Thr Gly Val Lys Pro Ala Ser Gly Asp Ser Pro Leu Gln Leu Gly
           275                 280                 285
   cca aag gaa gag gac gta gag aag cct gtc aac aac ccc ttc tcc ttc      912
   Pro Lys Glu Glu Asp Val Glu Lys Pro Val Asn Asn Pro Phe Ser Phe
       290                 295                 300
   ttg gga cgc ttc ctc ctg gga act cta gca gct gcc tgg ttt gtg tta      960
   Leu Gly Arg Phe Leu Leu Gly Thr Leu Ala Ala Ala Trp Phe Val Leu
   305                 310                 315                 320
   atc cct atc tac atg tgg atc aag gat cag atc gtt ccc aaa gac caa     1008
   Ile Pro Ile Tyr Met Trp Ile Lys Asp Gln Ile Val Pro Lys Asp Gln
                   325                 330                 335
   ccc atc tga                                                         1017
   Pro Ile
<210> 4
   <211> 338
   <212> PRT
   <213> Arabidopsis thaliana
<400> 4
   Met Ala Ser Leu Met Leu Asn Gly Ala Ile Thr Phe Pro Lys Gly Leu
   1               5                   10                  15
   Gly Ser Pro Gly Ser Asn Leu His Ala Lys Ser Ile Pro Arg Pro Thr
               20                  25                  30
   Leu Leu Ser Val Thr Arg Thr Ser Thr Pro Arg Leu Ser Val Ala Thr
           35                  40                  45
   Lys Cys Ser Ser Ser Ser Val Ser Ser Ser Arg Pro Ser Ala Gln Pro
       50                  55                  60
   Arg Phe Ile Gln His Lys Lys Glu Ala Tyr Trp Phe Tyr Arg Phe Leu
   65                  70                  75                  80
   Ser Ile Val Tyr Asp His Val Ile Asn Pro Gly His Trp Thr Glu Asp
                   85                  90                  95
   Met Arg Asp Asp Ala Leu Glu Pro Ala Asp Leu Ser His Pro Asp Met
   100 105 110
   Arg Val Val Asp Val Gly Gly Gly Thr Gly Phe Thr Thr Leu Gly Ile
   115 120 125
   Val Lys Thr Val Lys Ala Lys Asn Val Thr Ile Leu Asp Gln Ser Pro
   130 135 140
   His Gln Leu Ala Lys Ala Lys Gln Lys Glu Pro Leu Lys Glu Cys Lys
   145 150 155 160
   Ile Val Glu Gly Asp Ala Glu Asp Leu Pro Phe Pro Thr Asp Tyr Ala
   165 170 175
   Asp Arg Tyr Val Ser Ala Gly Ser Ile Glu Tyr Trp Pro Asp Pro Gln
   180 185 190
   Arg Gly Ile Arg Glu Ala Tyr Arg Val Leu Lys Ile Gly Gly Lys Ala
   195 200 205
   Cys Leu Ile Gly Pro Val Tyr Pro Thr Phe Trp Leu Ser Arg Phe Phe
   210 215 220
   Ser Asp Val Trp Met Leu Phe Pro Lys Glu Glu Glu Tyr Ile Glu Trp
   225 230 235 240
   Phe Lys Asn Ala Gly Phe Lys Asp Val Gln Leu Lys Arg Ile Gly Pro
   245 250 255
   Lys Trp Tyr Arg Gly Val Arg Arg His Gly Leu Ile Met Gly Cys Ser
   260 265 270
   Val Thr Gly Val Lys Pro Ala Ser Gly Asp Ser Pro Leu Gln Leu Gly
   275 280 285
   Pro Lys Glu Glu Asp Val Glu Lys Pro Val Asn Asn Pro Phe Ser Phe
   290 295 300
   Leu Gly Arg Phe Leu Leu Gly Thr Leu Ala Ala Ala Trp Phe Val Leu
   305 310 315 320
   Ile Pro Ile Tyr Met Trp Ile Lys Asp Gln Ile Val Pro Lys Asp Gln
   325 330 335
   Pro Ile
<210> 5
   <211> 774
   <212> DNA
   <213> Arabidopsis thaliana
<220>
   <221> CDS
   <222> (1)..(774)
   <223>
<400> 5
   tgc agc agc agc agc gtg tcg tct tcc cgg cca tcg gcg caa cct agg      48
   Cys Ser Ser Ser Ser Val Ser Ser Ser Arg Pro Ser Ala Gln Pro Arg
   1               5                   10                  15
   ttc att cag cac aag aag gag gct tac tgg ttc tac agg ttc tta tcc      96
   Phe Ile Gln His Lys Lys Glu Ala Tyr Trp Phe Tyr Arg Phe Leu Ser
               20                  25                  30
   atc gta tac gac cat gtc atc aat cct ggg cat tgg acc gag gat atg     144
   Ile Val Tyr Asp His Val Ile Asn Pro Gly His Trp Thr Glu Asp Met
           35                  40                  45
   aga gac gac gct ctt gag cca gcg gat ctc agc cat ccg gac atg cga     192
   Arg Asp Asp Ala Leu Glu Pro Ala Asp Leu Ser His Pro Asp Met Arg
       50                  55                  60
   gtg gtc gat gtc ggc ggc gga act ggt ttc act act ctg ggc ata gtc     240
   Val Val Asp Val Gly Gly Gly Thr Gly Phe Thr Thr Leu Gly Ile Val
   65                  70                  75                  80
   aag aca gtg aag gcc aag aat gtg acc att ctg gac cag tcg cca cat     288
   Lys Thr Val Lys Ala Lys Asn Val Thr Ile Leu Asp Gln Ser Pro His
                   85                  90                  95
   cag ctg gcc aaa gca aag caa aag gag ccg ttg aaa gaa tgc aag atc     336
   Gln Leu Ala Lys Ala Lys Gln Lys Glu Pro Leu Lys Glu Cys Lys Ile
               100                 105                 110
   gtc gag gga gat gct gag gat ctt cct ttt cca acc gat tat gct gac     384
   Val Glu Gly Asp Ala Glu Asp Leu Pro Phe Pro Thr Asp Tyr Ala Asp
           115                 120                 125
   aga tac gtt tct gct gga agc att gag tac tgg ccg gac ccg cag agg     432
   Arg Tyr Val Ser Ala Gly Ser Ile Glu Tyr Trp Pro Asp Pro Gln Arg
       130                 135                 140
   gga ata agg gaa gcg tac agg gtt ctc aag atc ggt ggc aaa gcg tgt     480
   Gly Ile Arg Glu Ala Tyr Arg Val Leu Lys Ile Gly Gly Lys Ala Cys
   145                 150                 155                 160
   ctc atc ggc cct gtc tac cca acc ttc tgg ctc tct cgc ttc ttt tct     528
   Leu Ile Gly Pro Val Tyr Pro Thr Phe Trp Leu Ser Arg Phe Phe Ser
                   165                 170                 175
   gat gtc tgg atg ctc ttc ccc aag gag gaa gag tac att gag tgg ttc      576
   Asp Val Trp Met Leu Phe Pro Lys Glu Glu Glu Tyr Ile Glu Trp Phe
               180                 185                 190
   aag aat gcc ggt ttc aag gac gtt cag ctc aag agg att ggc ccc aag      624
   Lys Asn Ala Gly Phe Lys Asp Val Gln Leu Lys Arg Ile Gly Pro Lys
           195                 200                 205
   tgg tac cgt ggt gtt cgc agg cac ggc ctt atc atg gga tgt tct gtc      672
   Trp Tyr Arg Gly Val Arg Arg His Gly Leu Ile Met Gly Cys Ser Val
       210                 215                 220
   act ggt gtt aaa cct gcc tcc ggt gat tct cct ctc cag ctt ggt cca      720
   Thr Gly Val Lys Pro Ala Ser Gly Asp Ser Pro Leu Gln Leu Gly Pro
   225                 230                 235                 240
   aag gaa gag gac gta gag aag cct gtc aac aac ccc ttc tcc ttc ttg      768
   Lys Glu Glu Asp Val Glu Lys Pro Val Asn Asn Pro Phe Ser Phe Leu
                   245                 250                 255
   gga cgc                                                              774
   Gly Arg
<210> 6
   <211> 258
   <212> PRT
   <213> Arabidopsis thaliana
<400> 6
   Cys Ser Ser Ser Ser Val Ser Ser Ser Arg Pro Ser Ala Gln Pro Arg
   1               5                   10                  15
   Phe Ile Gln His Lys Lys Glu Ala Tyr Trp Phe Tyr Arg Phe Leu Ser
               20                  25                  30
   Ile Val Tyr Asp His Val Ile Asn Pro Gly His Trp Thr Glu Asp Met
           35                  40                  45
   Arg Asp Asp Ala Leu Glu Pro Ala Asp Leu Ser His Pro Asp Met Arg
       50                  55                  60
   Val Val Asp Val Gly Gly Gly Thr Gly Phe Thr Thr Leu Gly Ile Val
   65                  70                  75                  80
   Lys Thr Val Lys Ala Lys Asn Val Thr Ile Leu Asp Gln Ser Pro His
                   85                  90                  95
   Gln Leu Ala Lys Ala Lys Gln Lys Glu Pro Leu Lys Glu Cys Lys Ile
               100                 105                 110
   Val Glu Gly Asp Ala Glu Asp Leu Pro Phe Pro Thr Asp Tyr Ala Asp
           115                 120                 125
   Arg Tyr Val Ser Ala Gly Ser Ile Glu Tyr Trp Pro Asp Pro Gln Arg
       130                 135                 140
   Gly Ile Arg Glu Ala Tyr Arg Val Leu Lys Ile Gly Gly Lys Ala Cys
   145                 150                 155                 160
   Leu Ile Gly Pro Val Tyr Pro Thr Phe Trp Leu Ser Arg Phe Phe Ser
                   165                 170                 175
   Asp Val Trp Met Leu Phe Pro Lys Glu Glu Glu Tyr Ile Glu Trp Phe
               180                 185                 190
   Lys Asn Ala Gly Phe Lys Asp Val Gln Leu Lys Arg Ile Gly Pro Lys
           195                 200                 205
   Trp Tyr Arg Gly Val Arg Arg His Gly Leu Ile Met Gly Cys Ser Val
       210                 215                 220
   Thr Gly Val Lys Pro Ala Ser Gly Asp Ser Pro Leu Gln Leu Gly Pro
   225                 230                 235                 240
   Lys Glu Glu Asp Val Glu Lys Pro Val Asn Asn Pro Phe Ser Phe Leu
                   245                 250                 255
   Gly Arg
<210> 7
   <211> 768
   <212> DNA
   <213> Arabidopsis thaliana
<220>
   <221> CDS
   <222> (1)..(768)
   <223>
<400> 7
   agc agc agc gtg tcg tct tcc cgg cca tcg gcg caa cct agg ttc att 48
   Ser Ser Ser Val Ser Ser Ser Arg Pro Ser Ala Gln Pro Arg Phe Ile
   1 5 10 15
   cag cac aag aag gag gct tac tgg ttc tac agg ttc tta tcc atc gta 96
   Gln His Lys Lys Glu Ala Tyr Trp Phe Tyr Arg Phe Leu Ser Ile Val
   20 25 30
   tac gac cat gtc atc aat cct ggg cat tgg acc gag gat atg aga gac 144
   Tyr Asp His Val Ile Asn Pro Gly His Trp Thr Glu Asp Met Arg Asp
   35 40 45
   gac gct ctt gag cca gcg gat ctc agc cat ccg gac atg cga gtg gtc 192
   Asp Ala Leu Glu Pro Ala Asp Leu Ser His Pro Asp Met Arg Val Val
   50 55 60
   gat gtc ggc ggc gga act ggt ttc act act ctg ggc ata gtc aag aca 240
   Asp Val Gly Gly Gly Thr Gly Phe Thr Thr Leu Gly Ile Val Lys Thr
   65 70 75 80
   gtg aag gcc aag aat gtg acc att ctg gac cag tcg cca cat cag ctg 288
   Val Lys Ala Lys Asn Val Thr Ile Leu Asp Gln Ser Pro His Gln Leu
   85 90 95
   gcc aaa gca aag caa aag gag ccg ttg aaa gaa tgc aag atc gtc gag 336
   Ala Lys Ala Lys Gln Lys Glu Pro Leu Lys Glu Cys Lys Ile Val Glu
   100 105 110
   gga gat gct gag gat ctt cct ttt cca acc gat tat gct gac aga tac 384
   Gly Asp Ala Glu Asp Leu Pro Phe Pro Thr Asp Tyr Ala Asp Arg Tyr
   115 120 125
   gtt tct gct gga agc att gag tac tgg ccg gac ccg cag agg gga ata 432
   Val Ser Ala Gly Ser Ile Glu Tyr Trp Pro Asp Pro Gln Arg Gly Ile
   130 135 140
   agg gaa gcg tac agg gtt ctc aag atc ggt ggc aaa gcg tgt ctc atc 480
   Arg Glu Ala Tyr Arg Val Leu Lys Ile Gly Gly Lys Ala Cys Leu Ile
   145 150 155 160
   ggc cct gtc tac cca acc ttc tgg ctc tct cgc ttc ttt tct gat gtc 528
   Gly Pro Val Tyr Pro Thr Phe Trp Leu Ser Arg Phe Phe Ser Asp Val
   165 170 175
   tgg atg ctc ttc ccc aag gag gaa gag tac att gag tgg ttc aag aat 576
   Trp Met Leu Phe Pro Lys Glu Glu Glu Tyr Ile Glu Trp Phe Lys Asn
   180 185 190
   gcc ggt ttc aag gac gtt cag ctc aag agg att ggc ccc aag tgg tac 624
   Ala Gly Phe Lys Asp Val Gln Leu Lys Arg Ile Gly Pro Lys Trp Tyr
   195 200 205
   cgt ggt gtt cgc agg cac ggc ctt atc atg gga tgt tct gtc act ggt 672
   Arg Gly Val Arg Arg His Gly Leu Ile Met Gly Cys Ser Val Thr Gly
   210 215 220
   gtt aaa cct gcc tcc ggt gat tct cct ctc cag ctt ggt cca aag gaa 720
   Val Lys Pro Ala Ser Gly Asp Ser Pro Leu Gln Leu Gly Pro Lys Glu
   225 230 235 240
   gag gac gta gag aag cct gtc aac aac ccc ttc tcc ttc ttg gga cgc 768
   Glu Asp Val Glu Lys Pro Val Asn Asn Pro Phe Ser Phe Leu Gly Arg
   245 250 255
<210> 8
   <211> 256
   <212> PRT
   <213> Arabidopsis thaliana
<400> 8
   Ser Ser Ser Val Ser Ser Ser Arg Pro Ser Ala Gln Pro Arg Phe Ile
   1               5                   10                  15
   Gln His Lys Lys Glu Ala Tyr Trp Phe Tyr Arg Phe Leu Ser Ile Val
               20                  25                  30
   Tyr Asp His Val Ile Asn Pro Gly His Trp Thr Glu Asp Met Arg Asp
           35                  40                  45
   Asp Ala Leu Glu Pro Ala Asp Leu Ser His Pro Asp Met Arg Val Val
       50                  55                  60
   Asp Val Gly Gly Gly Thr Gly Phe Thr Thr Leu Gly Ile Val Lys Thr
   65                  70                  75                  80
   Val Lys Ala Lys Asn Val Thr Ile Leu Asp Gln Ser Pro His Gln Leu
                   85                  90                  95
   Ala Lys Ala Lys Gln Lys Glu Pro Leu Lys Glu Cys Lys Ile Val Glu
               100                 105                 110
   Gly Asp Ala Glu Asp Leu Pro Phe Pro Thr Asp Tyr Ala Asp Arg Tyr
           115                 120                 125
   Val Ser Ala Gly Ser Ile Glu Tyr Trp Pro Asp Pro Gln Arg Gly Ile
       130                 135                 140
   Arg Glu Ala Tyr Arg Val Leu Lys Ile Gly Gly Lys Ala Cys Leu Ile
   145                 150                 155                 160
   Gly Pro Val Tyr Pro Thr Phe Trp Leu Ser Arg Phe Phe Ser Asp Val
                   165                 170                 175
   Trp Met Leu Phe Pro Lys Glu Glu Glu Tyr Ile Glu Trp Phe Lys Asn
               180                 185                 190
   Ala Gly Phe Lys Asp Val Gln Leu Lys Arg Ile Gly Pro Lys Trp Tyr
           195                 200                 205
   Arg Gly Val Arg Arg His Gly Leu Ile Met Gly Cys Ser Val Thr Gly
       210                 215                 220
   Val Lys Pro Ala Ser Gly Asp Ser Pro Leu Gln Leu Gly Pro Lys Glu
   225                 230                 235                 240
   Glu Asp Val Glu Lys Pro Val Asn Asn Pro Phe Ser Phe Leu Gly Arg
                   245                 250                 255
<210> 9
   <211> 910
   <212> DNA
   <213> Nicotiana tabacum
<220>
   <221> CDS
   <222> (1)..(600)
   <223>
<400> 9
   gcg gcc gct gat caa tca cct cat caa ctt gcc aag gct aga gaa aag       48
   Ala Ala Ala Asp Gln Ser Pro His Gln Leu Ala Lys Ala Arg Glu Lys
   1               5                   10                  15
   gaa cct ttg aaa gaa tgt aag ata ttg gaa gga gat gct gag gat ttg       96
   Glu Pro Leu Lys Glu Cys Lys Ile Leu Glu Gly Asp Ala Glu Asp Leu
               20                  25                  30
   cct ttt cct act gat act ctt gat aga tat gtt tct gct gga ggc att      144
   Pro Phe Pro Thr Asp Thr Leu Asp Arg Tyr Val Ser Ala Gly Gly Ile
           35                  40                  45
   gag tat tgg ccc gat cca cag cgc ggt atc aag gaa gca tac cga gta      192
   Glu Tyr Trp Pro Asp Pro Gln Arg Gly Ile Lys Glu Ala Tyr Arg Val
       50                  55                  60
   ctg acc ata ggt ggt gtt gcc tgc tta ata ggt cct gtg tac ccg acg      240
   Leu Thr Ile Gly Gly Val Ala Cys Leu Ile Gly Pro Val Tyr Pro Thr
   65                  70                  75                  80
   ttt tgg cta tct cgt ttc ttt gca gat atg tgg atg ctc ttt cca aaa      288
   Phe Trp Leu Ser Arg Phe Phe Ala Asp Met Trp Met Leu Phe Pro Lys
                   85                  90                  95
   gaa gaa gaa tat ata gaa tgg ttc aaa aaa gct ggt ttc gct caa gtt      336
   Glu Glu Glu Tyr Ile Glu Trp Phe Lys Lys Ala Gly Phe Ala Gln Val
               100                 105                 110
   aaa ctc aag agg att ggc cca aaa tgg tat cgt ggt gtc tgt cgc cat      384
   Lys Leu Lys Arg Ile Gly Pro Lys Trp Tyr Arg Gly Val Cys Arg His
           115                 120                 125
   ggc ttg atc atg ggt tgt tct gtg act ggt gtc aag cca tat ttt ggg      432
   Gly Leu Ile Met Gly Cys Ser Val Thr Gly Val Lys Pro Tyr Phe Gly
       130                 135                 140
   gaa tct ccg ttg cag ctc ggt ccg aag gtt gag gat gtg agc aag cct      480
   Glu Ser Pro Leu Gln Leu Gly Pro Lys Val Glu Asp Val Ser Lys Pro
   145                 150                 155                 160
   gta aac cca ttc gta ttt ctc gtg cga ttc ctc ctt ggc ata act gct      528
   Val Asn Pro Phe Val Phe Leu Val Arg Phe Leu Leu Gly Ile Thr Ala
                   165                 170                 175
   gca act tat tac gtg ctc gtt cca ata tac atg tgg ctc aag gat caa      576
   Ala Thr Tyr Tyr Val Leu Val Pro Ile Tyr Met Trp Leu Lys Asp Gln
               180                 185                 190
   atc acc ccg aaa ggt cag cca atc tgaacaataa gaagaacgtc aatccaaaga      630
   Ile Thr Pro Lys Gly Gln Pro Ile
           195                 200
   gaagctctcc aagcattctg tttgagagta caccagtgac cacaaatcta tcacggaaca      690
   agaaagtttt tggcgtcgtt gcaagggtga atttgttgct ttagtttgtt agttttgcag      750
   ccttagaaag ggccttttgt aaagtttaat ttcatggtaa aacctagaaa tcattgtgac      810
   tattttctag ttgtataatc tatcagtcat gttcttttat cacgagttga gaaaactcgt      870
   cgaaataaat accagtaata cgttatttgc cagcggccgc                          910
<210> 10
   <211> 200
   <212> PRT
   <213> Nicotiana tabacum
<400> 10
   Ala Ala Ala Asp Gln Ser Pro His Gln Leu Ala Lys Ala Arg Glu Lys
   1               5                   10                  15
   Glu Pro Leu Lys Glu Cys Lys Ile Leu Glu Gly Asp Ala Glu Asp Leu
               20                  25                  30
   Pro Phe Pro Thr Asp Thr Leu Asp Arg Tyr Val Ser Ala Gly Gly Ile
           35                  40                  45
   Glu Tyr Trp Pro Asp Pro Gln Arg Gly Ile Lys Glu Ala Tyr Arg Val
       50                  55                  60
   Leu Thr Ile Gly Gly Val Ala Cys Leu Ile Gly Pro Val Tyr Pro Thr
   65                  70                  75                  80
   Phe Trp Leu Ser Arg Phe Phe Ala Asp Met Trp Met Leu Phe Pro Lys
                   85                  90                  95
   Glu Glu Glu Tyr Ile Glu Trp Phe Lys Lys Ala Gly Phe Ala Gln Val
               100                 105                 110
   Lys Leu Lys Arg Ile Gly Pro Lys Trp Tyr Arg Gly Val Cys Arg His
           115                 120                 125
   Gly Leu Ile Met Gly Cys Ser Val Thr Gly Val Lys Pro Tyr Phe Gly
       130                 135                 140
   Glu Ser Pro Leu Gln Leu Gly Pro Lys Val Glu Asp Val Ser Lys Pro
   145                 150                 155                 160
   Val Asn Pro Phe Val Phe Leu Val Arg Phe Leu Leu Gly Ile Thr Ala
                   165                 170                 175
   Ala Thr Tyr Tyr Val Leu Val Pro Ile Tyr Met Trp Leu Lys Asp Gln
               180                 185                 190
   Ile Thr Pro Lys Gly Gln Pro Ile
           195                 200
<210> 11
   <211> 16
   <212> DNA
   <213> Artificial sequence
<220>
   <223> Primer
<400> 11
   agaattcgcg gccgct 16
<210> 12
   <211> 32
   <212> DNA
   <213> Artificial sequence
<220>
   <223> Primer
<400> 12
   ctcatgcggc cgcgcgcaac gcaattaatg tg 32
<210> 13
   <211> 32
   <212> DNA
   <213> Artificial sequence
<220>
   <223> Primer
<400> 13
   tcatgcggcc gcgagatcca gttcgatgta ac 32
<210> 14
   <211> 21
   <212> DNA
   <213> Artificial sequence
<220>
   <223> Primer
<400> 14
   gtggattgat gtgatatctc c 21
<210> 15
   <211> 21
   <212> DNA
   <213> Artificial sequence
<220>
   <223> Primer
<400> 15
   gtaaggatct gagctacaca t 21
<210> 16
   <211> 22
   <212> DNA
   <213> Artificial sequence
<220>
   <223> Primer
<400> 16
   atggcctctt tgatgctcaa cg 22
<210> 17
   <211> 22
   <212> DNA
   <213> Artificial sequence
<220>
   <223> Primer
<400> 17
   gatgggttgg tctttgggaa cg 22
<210> 18
   <211> 22
   <212> DNA
   <213> Artificial sequence
<220>
   <223> Primer
<400> 18
   cctcttctca acttggtgga tc 22
<210> 19
   <211> 24
   <212> DNA
   <213> Artificial sequence
<220>
   <223> Primer
<400> 19
   ggggtttaca atgatacaat gatc 24
<210> 20
   <211> 18
   <212> DNA
   <213> Artificial sequence
<220>
   <223> Primer
<400> 20
   atgagcagca gcgtgtcg 18
<210> 21
   <211> 20
   <212> DNA
   <213> Artificial sequence
<220>
   <223> Primer
<400> 21
   gcgtcccaag aaggagaagg 20
<210> 22
   <211> 18
   <212> DNA
   <213> Artificial sequence
<220>
   <223> Primer
<400> 22
   atgtgcagca gcagcagc 18
<210> 23
   <211> 20
   <212> DNA
   <213> Artificial sequence
<220>
   <223> Primer
<400> 23
   gcgtcccaag aaggagaagg 20
<210> 24
   <211> 18
   <212> DNA
   <213> Artificial sequence
<220>
   <223> Primer
<400> 24
   atgtgcagca gcagcagc 18
<210> 25
   <211> 19
   <212> DNA
   <213> Artificial sequence
<220>
   <223> Primer
<400> 25
   tcagcgtccc aagaaggag 19
<210> 26
   <211> 18
   <212> DNA
   <213> Artificial sequence
<220>
   <223> Primer
<400> 26
   atgagcagca gcgtgtcg 18
<210> 27
   <211> 20
   <212> DNA
   <213> Artificial sequence
<220>
   <223> Primer
<400> 27
   tcagatgggt tggtctttgg 20
<210> 28
   <211> 18
   <212> DNA
   <213> Artificial sequence
<220>
   <223> Primer
<400> 28
   atgagcagca gcgtgtcg 18
<210> 29
   <211> 19
   <212> DNA
   <213> Artificial sequence
<220>
   <223> Primer
<400> 29
   gatgggttgg tctttggga 19
<210> 30
   <211> 18
   <212> DNA
   <213> Artificial sequence
<220>
   <223> Primer
<400> 30
   atgtgcagca gcagcagc 18
<210> 31
   <211> 19
   <212> DNA
   <213> Artificial sequence
<220>
   <223> Primer
<400> 31
   gatgggttgg tctttggga 19
<210> 32
   <211> 18
   <212> DNA
   <213> Artificial sequence
<220>
   <223> Primer
<400> 32
   atgagcagca gcgtgtcg 18
<210> 33
   <211> 19
   <212> DNA
   <213> Artificial sequence
<220>
   <223> Primer
<400> 33
   tcagcgtccc aagaaggag 19
<210> 34
   <211> 18
   <212> DNA
   <213> Artificial sequence
<220>
   <223> Primer
<400> 34
   atgtgcagca gcagcagc 18
<210> 35
   <211> 21
   <212> DNA
   <213> Artificial sequence
<220>
   <223> Primer
<400> 35
   gaaggatcag atgggttggt c 21